# EUROPEAN PATENT SPECIFICATION

(11) **EP 2 731 422 B1**
(45) Date of publication and mention of the grant of the patent: **09.08.2017**
(21) Application number: 12811507.8
(22) Date of filing: 29.06.2012
(51) Int. Cl.: C12N 15/113, A61P 25/00, C07K 14/475

(54) **miRNA antagonists for use in treating neurodegenerative disorders by increasing expression of GDNF**
miRNA Antagonisten zur Behandlung neurodegenerativer Krankheiten mittels erhöhter GDNF Expression
Antagonistes de miARN par traitement des maladies neurodégéneratives au moyen d'expression enlevé de GDNF

(30) Priority: 12.07.2011 US 201161506803 P
(43) Date of publication of application: 21.05.2014
(73) Proprietor: Saarma, Mart, 00570 Helsinki (FI); Andressoo, Jaan-Olle, 00810 Helsinki (FI)
(72) Inventor: Saarma, Mart, 00570 Helsinki (FI); Andressoo, Jaan-Olle, 00810 Helsinki (FI)
(74) Representative: Seppo Laine Oy
(86) International application number: PCT/FI2012/050695
(87) International publication number: WO 2013/007874

(56) References cited:
- EP-A1- 2 611 918
- WO-A1-95/29244
- WO-A1-99/36102
- WO-A1-2011/010737
- WO-A1-2013/055865
- WO-A2-2007/044937
- WO-A2-2007/070483
- WO-A2-2008/024499
- WO-A2-2008/091703
- WO-A2-2010/064248
- WO-A2-2010/093904
- WO-A2-2010/093906
- WO-A2-2012/036433
- W. J. LUKIW ET AL: "An NF- B-sensitive Micro RNA-146a-mediated Inflammatory Circuit in Alzheimer Disease and in Stressed Human Brain Cells", JOURNAL OF BIOLOGICAL CHEMISTRY, vol. 283, no. 46, 14 November 2008 (2008-11-14), pages 31315-31322, XP055152249, ISSN: 0021-9258, DOI: 10.1074/jbc.M805371200
- DATABASE WPI Week 201111 Thomson Scientific, London, GB; AN 2011-B10853 XP002732344, -& WO 2011/010737 A1 (KYOWA HAKKO KIRIN CO LTD) 27 January 2011 (2011-01-27)
- DORSETT Y. ET AL.: 'MicroRNA-155 Suppresses Activation- Induced Cytidine Deaminase-Mediated Myc-Igh Translocation' IMMUNITY vol. 28, 2008, pages 630 - 638, XP055084162
- KAKOKI M. ET AL.: 'Altering the Expression in Mice of Genes by Modifying Their 3' REGIONS, 2004, DEVELOPMENTAL CELL vol. 6, April 2004, pages 597 - 606, XP055137714
- GIRARD B. M. ET AL.: 'PACAP/VIP and Receptor Characterization in Micturition Pathways in Mice with Overexpression of NGF in Urothelium' JOURNAL OF MOLECULAR NEUROSCIENCE vol. 42, 2010, pages 378 - 389, XP055137716
- EBERT M. S. ET AL.: 'MicroRNA sponges: Progress and possibilities' RNA vol. 16, 2010, pages 2043 - 2050, XP002656479
- ROSHAN R. ET AL.: 'MicroRNAs: novel therapeutic targets in neurodegenerative diseases' DRUG DISCOVERY TODAY vol. 14, no. 23-24, 2009, pages 1123 - 1129, XP026965404
- LEE S-T. ET AL.: 'Altered microRNA regulation in Huntington's disease models' EXPERIMENTAL NEUROLOGY vol. 227, 2011, pages 172 - 179, XP027585906
- BARTEL D. P.: 'MicroRNAs: Target Recognition and Regulatory Functions' CELL vol. 136, 2009, pages 215 - 233, XP055011377
- FRIEDMAN L. M. ET AL.: 'MicroRNAs are essential for development and function of inner ear hair cells in vertebrates' PROCEEDINGS OF THE NATIONAL ACADEMY OF SCIENCES OF THE UNITED STATES OF AMERICA vol. 106, no. 19, 2009, pages 7915 - 7920, XP055137721
- LAU P. ET AL.: 'Dysregulated microRNAs in neurodegenerative disorders' SEMINARS IN CELL & DEVELOPMENTAL BIOLOGY vol. 21, 2010, pages 768 - 773, XP027264906
- MAKHANOVA N. ET AL.: 'Salt-Sensitive Blood Pressure in Mice With Increased Expression of Aldosterone Synthase' HYPERTENSION vol. 51, 2008, pages 134 - 140, XP055137724
- LI W.: 'Muscle-derived but not centrally derived transgene GDNF is neuroprotective in G93A-SOD1 mouse model of ALS' EXPERIMENTAL NEUROLOGY vol. 203, 2007, pages 457 - 471, XP005737587
- XIE Y. ET AL.: 'BDNF Overexpression in the Forebrain Rescues Huntington's Disease Phenotypes in YAC128' MICE vol. 30, no. 44, 2010, pages 14708 - 14718, XP055137725
- MELLIOS N. ET AL.: 'A set of differentially expressed miRNAs, including miR-30a-5p, act as post-transcriptional inhibitors of BDNF in prefrontal cortex' HUMAN MOLECULAR GENETICS vol. 17, no. 19, 2008, pages 3030 - 3042, XP055053999

## Description

### FIELD OF THE INVENTION

The present specification relates to the fields of knockout (KO) animal production. The specification is directed to a transgenic KO animal comprising a heterozygous or homozygous deletion or functional deletion of the gene's native 3' untranslated region (3'UTR) at least in one of its endogenous gene loci, wherein the disrupted endogenous gene is transcribed into an mRNA without its native 3'UTR. Instead, a 3'UTR of choice, knocked in by the experimenter, is transcribed into an mRNA. The 3'UTR KO animals provide a new approach to study gene function as they enable to overexpress the gene products what are negatively regulated via their 3'UTR-s exclusively in those cells that already transcribe the gene, thereby avoiding the misexpression problem present in the animals produced by conventional transgenesis methods. The specification is further directed to KO animals, in which the gene with deletion of 3'UTR is glial cell line-derived neuritrophic factor (GDNF), nerve growth factor (NGF) or brain-derived neurotrophoc factor (BDNF).

### BACKGROUND OF THE INVENTION

Currently, the function of a gene *in vivo* is studied by either overexpressing it using a transgenic approach, or by knocking it out (KO). The main problem associated with transgenic overexpression using either cDNA or bacterial artificial chromosome based strategies is misexpression in space and time. The common bottlenecks in the knockout (KO) or conditional knockout (cKO) approach are the structural and/or functional homologues which may mask the effect of the studied gene, or the lethality of the KO animals. Therefore, a method that would enable to overexpress a gene product only in the natively expressing cells would benefit those fields of biology where genetically modified animal models are used.

Glial cell line-derived neurotrophic factor GDNF is a neurotrophic factor (NTF) that promotes axonal branching and survival of midbrain dopamine (DA) neurons that specifically degenerate in currently incurable Parkinson's disease (PD). GDNF and its close relative neurturin (NRTN) are clinically relevant and have been, and are at present, tested in clinical trials of PD with highly promising, but yet with somewhat conflicting outcomes highlighting a need for the better understanding of their biology in vivo and improved treatment strategies. Other well recognized members of the neurotrophin family include brain-derived neurotrophic factor (BDNF) and nerve growth factor (NGF).

Recently, it was experimentally shown that micro RNA-s (miR-s), about 20-22 bp single stranded RNA molecules, control the levels of hundreds of mammalian gene products by binding to the 3' UTRs of their target mRNA-s, effectively destabilizing them and/or suppressing their translation. Moreover, bioinformatics approaches predict that the expression levels of more than half of mammalian genes are controlled by miR-s¹. However, most often a 3'UTR is regulated by a combination of multiple miR-s, and a single miR is predicted to regulate over 100 mRNA-s, making it difficult to analyze the biological importance of miR regulation of a given gene product, particularly, in vivo.

The U.S. patent application US 20110086904 presents a method for enhancing the stability of an mRNA molecule by inserting a stability inducing motif at the 3'UTR of said mRNA molecule. This stability inducing motif is said to comprise a site specific deletion and substitution of a predetermined nucleotide sequence at the 3'UTR. Related to the present invention, the U.S. patent application US 20100267573 suggests GDNF, BDNF and NGF to be potential RNAi targets. The invention includes methods for in vivo identification of endogenous mRNA targets of miRNAs and for generating a gene expression profile of miRNAs present in mRNA-protein complexes, wherein said mRNA can encode a protein selected from the group including f. ex. BNDF, GDNF, and NGF. Similarly, the US-application US 20100167330, which illustrates micro mechanical devices for control of cell-cell interaction, suggests also knocking down of GDNF, BDNF, and NGF expression by RNAi.

There exist several examples of patent applications describing RNAi therapies related to the treatment of neurodegenerative diseases, said diseases including also PD, Alzheimer's disease, Huntington's disease, dementia, and ALS (US 20100132060, US 20100098664, US 20110039785, US 20110052666, US 20100249208, US 20100113351, US 20100048678, and US 20080279846). None of those applications, however, suggests that genes encoding GDNF, BDNF or NGF could be targets for such therapy. Furthermore, there exist numerous patent applications and patents relating to different gene therapies related to the treatment of PD or Alzheimer's disease (US 20060239966, US 20080145340, US 6800281, and US 6245330).

An article by Hutchison and Mattson (Aging, 2011; vol. 3:179-180) describes that previous research has demonstrated that miR-30a acts to functionally repress BDNF expression in the cortex. Up-regulation of BDNF by energy restriction (CR) has been shown to mediate, in part, the increased neurogenesis by CR and is also thought to play an important role in learning and memory. The regulation of BDNF by the miR-34 family is said to represent a potential avenue for miRNAs as mediators of effects of dietary energy intake on neuronal vulnerability in aging and disease. The article also mentions that it would be important to determine whether changes in the expression of miRNAs 34a, 30e, and 181a do in fact mediate effects of energy intake on neuronal vulnerability. This is possible to accomplish by overexpressing or knocking down each of these miRNAs in neurons of interest in animal models of Alzheimer's, Parkinson's, and Huntington's diseases.

WO 2008091703 relates generally to chemically modified oligonucleotides useful for modulating expression of microRNAs and pre-microRNAs. More particularly, WO 2008091703 discloses single stranded chemically modified oligonucleotides for inhibiting microRNA and pre-microRNA expression and methods of making and using the modified oligonucleotides. Also disclosed are compositions and methods for silencing microRNAs in the central nervous system.

WO 2011010737 discloses a guide nucleic acid for use in the cleavage of micro-RNA. WO 2011010737 also discloses antagonists of miRNAs which bind the 3'UTR of a gene for use in the treatment of a neurodegenerative disease.

WO2007044937 shows that antisense oligonucleotides, e.g. antagomirs or morpholinos complementary to miR-30a or miR-30b can be used in treating Alzheimer's or Parkinson's disease. A method of treating Alzheimer's disease by disruption of RISC activity mediated by anti-miRNA oligonucleotides, e.g. miR-1 or miR-206, is also disclosed.

WO 2010093906 discloses an antisense oligonucleotide being complementary to a naturally occurring antisense transcript of GDNF for use in treating a neurodegenerative disease, e.g., Alzheimer's disease, Parkinson's disease or ALS.

An article by Hebert and De Strooper (Trends in Neurosciences, 2009; vol. 32:199-206) illustrates potential problems related to the bioavailability and toxicity issues, and also to the blood-brain barrier possibly inhibiting the effective delivery of the drugs in the brain. The authors also point out that although initial studies in non-human primates have emphasized the potential for miRNA-based therapeutics, it should be taken into account that as a single miRNA can regulate hundreds of transcripts, systemic delivery of a miRNA mimetic or sponge may result in undesirable off-target and tissue specific effects.

Also some other articles describe possible problems related to using miR/antagomiR therapies. A scientific article by Sassen et al. (Virchows Arch, 2008; vol. 452:1-10) presents one limitation of antisense RNA therapies to be the restricted number of cells that can be targeted. Also any approach to knock down a particular miRNA with antisense oligonucleotides will only result in partial knockdown. The authors, however, admit that even a partial effect on function may be of therapeutic value in neurodegenerative diseases, such as Parkinson's or Alzheimer's disease. For example, a partial restoration of dopamine production by antisense therapy might result in a significant clinical improvement in Parkinson patients. Similarly, a partial reduction of the disease-causing proteins in Alzheimer's disease may lead to a clinical improvement and might be achievable by RNA based or miRNA gene therapy.

What is still needed in the art are transgenic animal models for studying the function of a gene in vivo when the gene is overexpressed. Currently, the main problem associated with transgenic overexpression is misexpression in space and time. The common bottlenecks in the KO or cKO approach are the structural and/or functional homologues which may mask the effect of the studied gene, or the lethality of the KO animals. Therefore, a method that would enable the overexpression of a gene product only in the natively expressing cells and without side-effects would be very beneficial for the fields of biology where genetically modified animal models are used.

### SUMMARY OF THE INVENTION

The present invention is defined in the claims.

The present invention is directed to a short interfering RNA (siRNA), double-stranded RNA (dsRNA), micro-RNA (miRNA), short hairpin RNA (shRNA), miRNA sponge, anti-miRNA, morpholino oligonucleotide, miRNA target site protector, or antisense oligonucleotide targeting miR-133a, miR-133b, miR-125a-5p, miR-125b-5p, miR-30a, miR-30b, miR-96, miR-9, and/or miR-146, for use in the treatment of Parkinson's disease, Alzheimer's disease, Huntington's disease, dementia, ALS, spinal cord injury, age associated memory decline, age related drop in physical activity, or age related decline in motorcoordination, wherein said treatment is characterized by increasing the expression level of GDNF in a cell expressing endogenous GDNF.

### BRIEF DESCRIPTION OF THE DRAWINGS

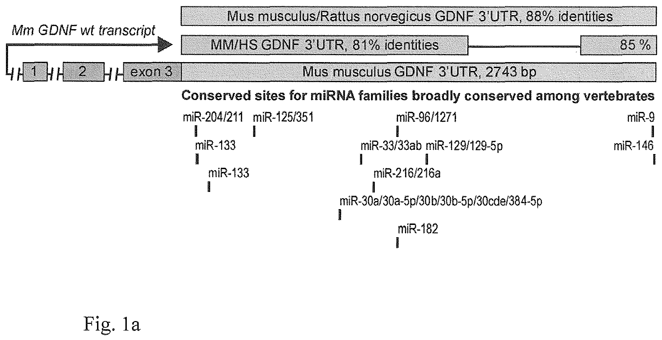

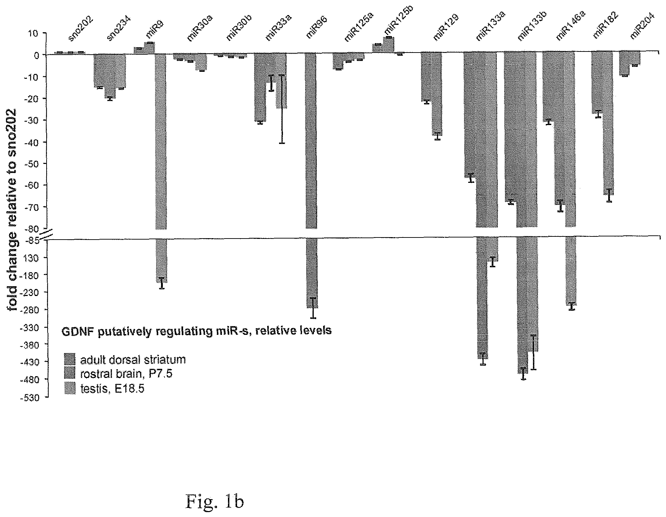

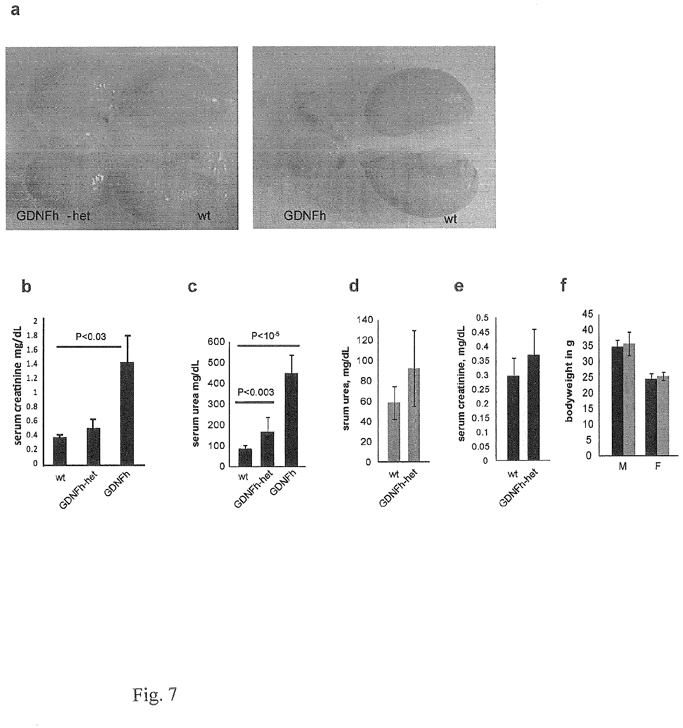

**Figure 1****.** GDNF 3'UTR is conserved and contains several putative binding sites for micro RNA-s (miRs). **a**, The 3'UTR of GDNF mRNA is conserved in evolution. Length of exons and 3'UTR is drawn in scale. Note the numbers of identities between human and rodent sequences and that the most conserved bioinformatics predicted miR binding sites cluster within the conserved areas (colored bars). Shown are micro RNA (miR) binding sites broadly conserved among vertebrates Source: Blast, TragetScan. **b**, Levels of mature miR-s relative to sno202 with the most conserved seed sequences in GDNF 3'UTR measured in tissues where GDNF levels are known,- or expected to have biological function. Note high levels of miR-9, a previously established highly abundant brain specific miR, in the brain verifying sensitivity of our measurements. Absence of the corresponding color coded bar represents lack of detectable expression (Cp>35), i.e. miR-96 was only found in the rostral brain at P7.5, miR-129 is absent in the testis, etc. Mature miR levels were assessed with TaqMan QPCR system, 2-4 experiments were performed with 3-5 wt animals analyzed per tissue. 3-4 biological repeats were run per miR, shown are pooled data from all experiments, error bars indicate SD.

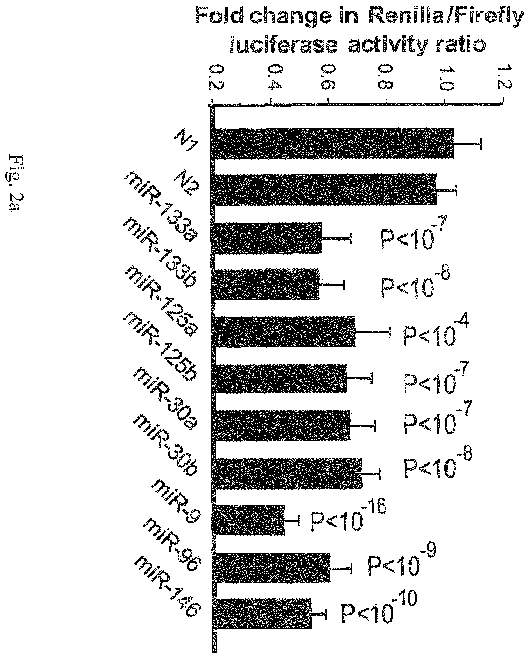

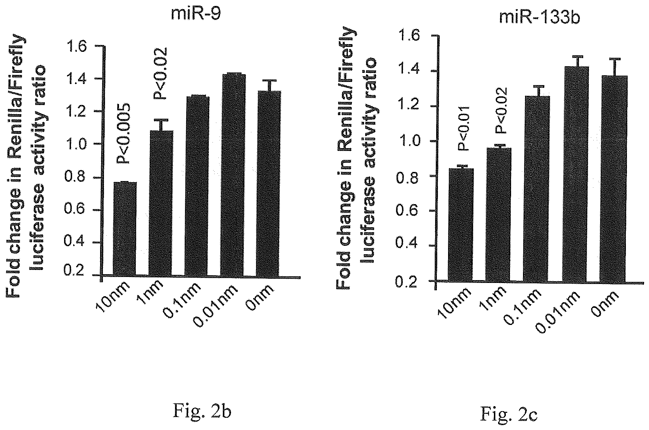

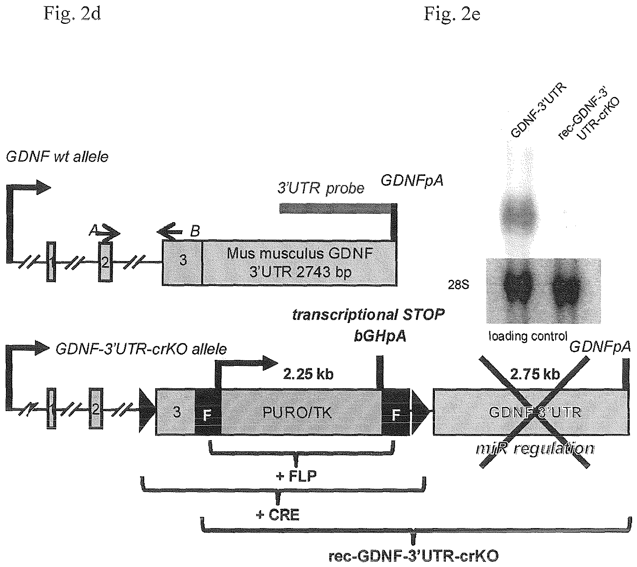

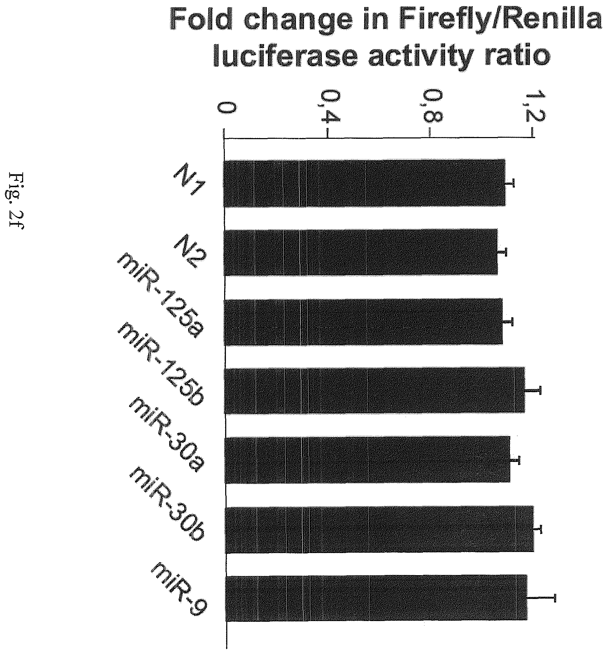

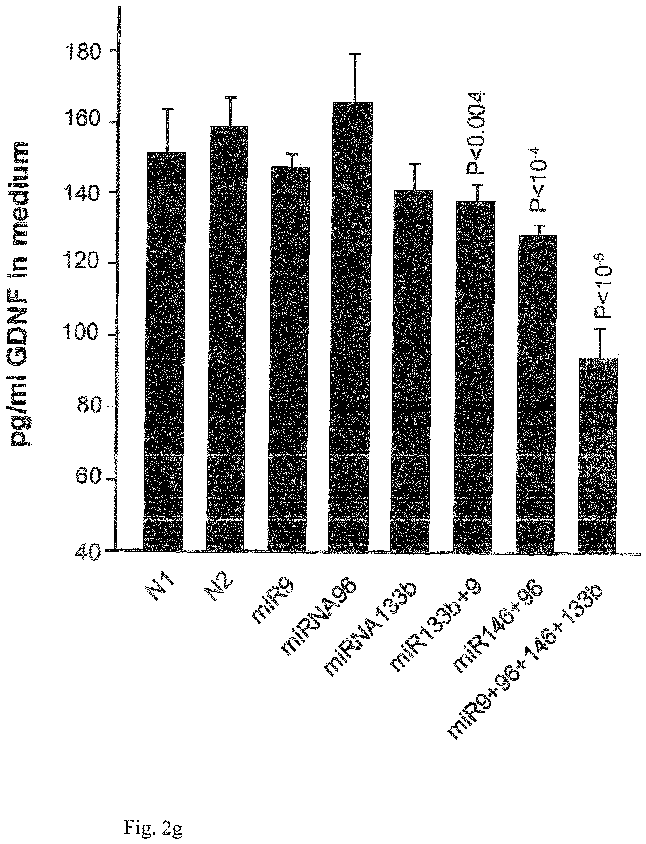

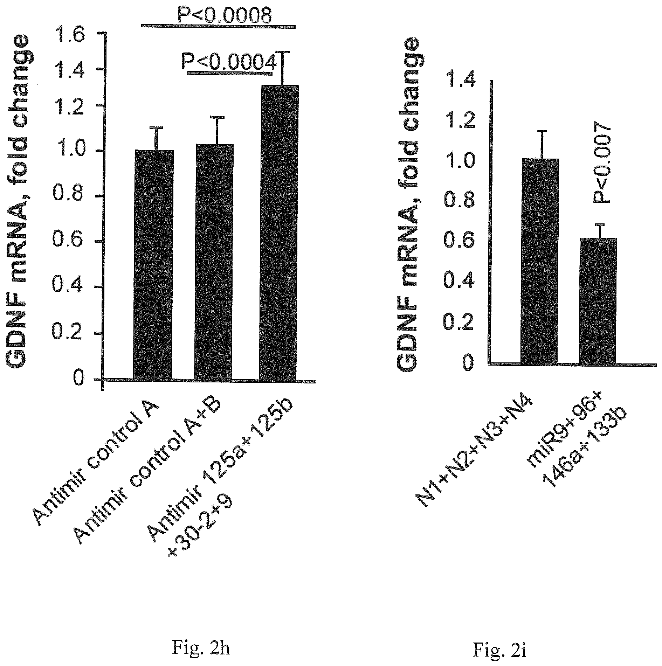

**Figure 2****.** miR regulation of GDNF 3'UTR in vitro and design of the conditionally reversible GDNF 3'UTR KO (GDNF-3'UTR-crKO) mice. **a**, GDNF 3'UTR was cloned from 129Ola BAC library (CHORI) into Dual-Luciferase Reporter Assay System (Promega) and co-transfected into HEK-293 cells either with negative control pre-miRs (N1, N2) or putative GDNF 3'UTR regulating pre-miR-s at 10nM final concentration as indicated. **b** and **c**, GDNF 3'UTR is suppressed by specific miR-s in a concentration dependent manner. For **a,b,c and f** 3 experiments with 3-4 repeats per miR per experiment was performed, error bars indicate SD, shown is pooled data from 3 experiments. **d**, For the substitution of native GDNF 3'UTR, a FRT flanked Puro/TK cassette containing a strong mammalian transcriptional stop signal (bovine growth hormone polyadenylation signal, bGHpA) was inserted after the GDNF's last, third exon. Crosses of such animals to mice expressing FLP recombinase result in excision of the Puro/TK cassette and restoration of transcription to GDNF 3'UTR. We called such an allele conditionally reversible 3'UTR knock out (3'UTR-crKO) allele. Crosses of such animals to mice expressing Cre recombinase would lead to deletion of GDNF exon 3 , what encodes for the mature GDNF protein, resulting in GDNF protein KO mice. **e**, to verify the 3'UTR crKO strategy in vitro, recombinant GDNF 3'UTR (rec-GDNF-3'UTR-crKO as indicated on **d**,) was cloned into Dual-Luciferase Reporter Assay System (Promega), transfected into HEK-293 cells and the transcript was analyzed with Northern blotting method using a probe complementary to GDNF 3'UTR as indicated on **d** with a blue line. **f**, As expected from **d** and **e,** analysis of rec-GDNF-3'UTR-crKO using a set of pre-miR-s from **a** revealed lack of miR mediated suppression. **g** and **i**, miR-s suppress endogenous GDNF protein and mRNA levels in an additive manner. U87 astrocytoma cells were co-transfected either with negative controls or indicated pre-miR-s, experiment was repeated 3 times with 2-3 repeats per experiment with 2-3 dilutions each. GDNF protein levels in the cell culture medium were assessed using ELISA method. Transfection of miR-s did not affect cellular survival of U87 cells (Suppl. Fig 1a). **h**, Anti-miR suppression of the most abundant GDNF regulating miR-s in U87 cells (Suppl. Fig. 1b) results in upregulation of GDNF mRNA levels. In **h** and **i**, GDNF mRNA was analysed with QPCR using primers A and B as indicated on **d,** from 4 identically treated wells, each well in triplicate in QPCR runs. For g, h and i representative experiments are shown, error bars indicate SD.

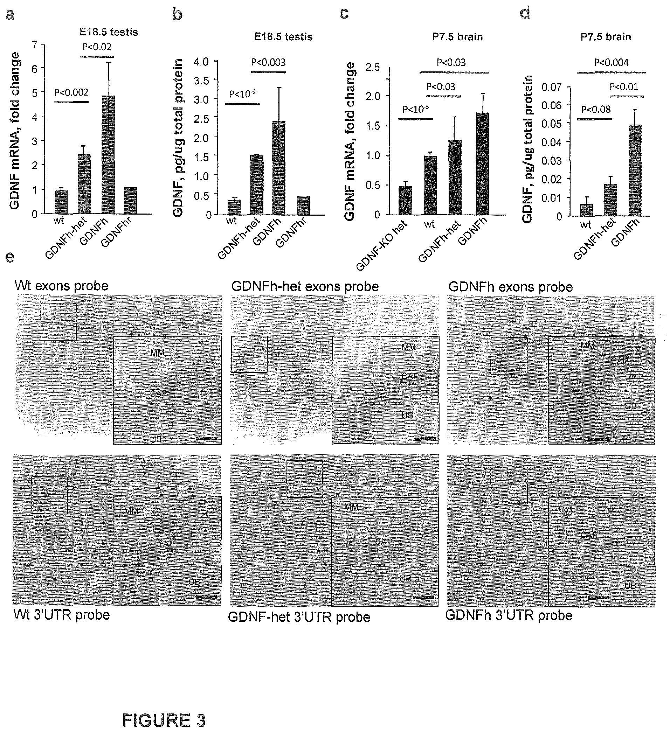

**Figure 3****.** In GDNF-3'UTR-crKO mice GDNF levels are elevated up to 10 fold in GDNF natively expressing cells. **a** and **c**, QPCR analysis of GDNF mRNA levels using primers A and B as indicated on Fig. 2d at embryonal day 18.5 (E18.5) in testis and post natal day 7.5 (P7.5) rostral brain. cDNA derived from heterozygous GDNF coding sequence KO animals obtained from crossing of the GDNF-3'UTR-crKO animals to Deleter-Cre mice as indicated on Fig. 2d served as controls of sensitivity of the QPCR setting since the heterozygous GDNF KO animals are known to harbor ca 2 fold less GDNF mRNA. GDNF protein levels from tissues were analysed with ELISA. Note that in the testis (**a**), GDNF mRNA levels are upregulated more prominently, than in the brain (**c**) whereas GDNF protein levels are similarily increased in both organs (**b** and **d**). Animals were designated GDNF hypermorphs (GDNFh). Restoration of transcription to wt GDNF 3'UTR by crosses of the GDNF-3'UTR-crKO animals to Deleter-FLP line resulted in restoration of the wt GDNF levels (left lanes on **a** and **b**), animals were designated GDNFh rescued (GDNFhr). 3-4 animals (except for the GDNFhr animals where 1-2 animals were used) were analyzed in 2-5 experiments each containing 2-4 replicates per material derived from an animal. Error bars indicate SD, graphs represent compilation from all experiments.
   **e**, In situ analysis of GDNF expression site at E10.5. GDNF mRNA levels are known to be expressed at high levels in a distinct structure called CAP condensate of the metanephric mesenchyme (MM) in the developing kidney making this structure especially suitable for assessing the precise site of GDNF expression. GDNF expression at E10 induces ureteric bud (UB) formation, the first step in kidney development. Note that probe complementary to GDNF exons as indicated with a red line on Fig. 7c. readily recognizes GDNF mRNA in whole mount stained vibratome sections from urogenital tracts from E10.5 GDNFh-het and GDNFh mice, suggesting elevated GDNF exons containing mRNA levels as compared to the wt littermate controls (upper panel). On the other hand, probe complementary to GDNF 3'UTR (Fig. 2d blue line), revelas about 2 fold reduced signal in GDNFh-het animals, and practically no signal in GDNFh mice, as expected (lower panel). Experiment was repeated 3 times, at least 3 animals were analyzed per genotype. Representative images are shown. Scale bar 10µm.

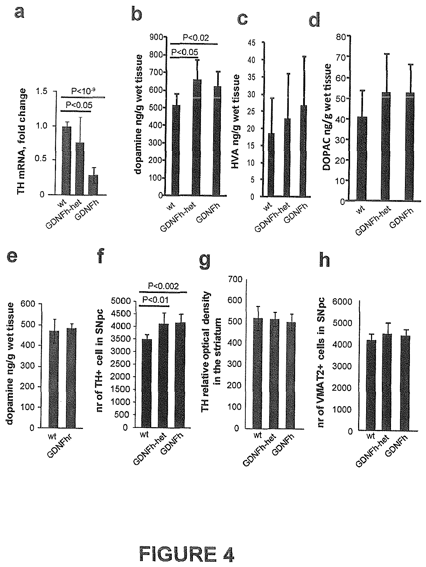

**Figure 4****.** Nigrostriatal dopamine system in one week old GDNF-3'UTR-crKO (GDNFh) mice. **a**, QPCR analysis of tyrosine hydroxylase (TH) mRNA levels in rostral brain at P7.5. At least 4 animals were analysed per genotype in 3 QPCR runs with 3 repeats per animal per run. Graph represents a combined result from all runs, error bars indicate SD. **b,c,d** high performance liquid chromatography (HPLC) analysis of rostral brain dopamine and its metabolite homovanillic acid (HVA) and 3,4-dihydroxyphenylacetic acid (DOPAC) levels. n=7 animals analyzed per genotype, error bars indicate SD. **e,** dopamine levels in GDNFhr are not elevated. Note that absolute dopamine values vary between experiments, likely reflecting differences in the size of the isolated rostral brain area. However, within the experiment, rostral brain isolation is performed identically for all pups. **f**, At P7.5 number of TH positive neurons in substantia nigra (SN) is increased in GDNFh-het and GDNFh animals, n=5-7 animals per genotype, cells were counted by blinded observer. **g**, optical density of TH striatal immunostaining, reflecting striatal TH levels and density of striatal dopaminergic innervations, is unchanged at P7.5 in GDNFh mice (n=7 animals per genotype). **h**, At P7.5 number of VMAT2 positive neurons in substantia nigra (SN) is increased in GDNFh-het and GDNFh animals. However, the increase is not statistically significant. n=7 animals per genotype, cells were counted by a blinded observer.

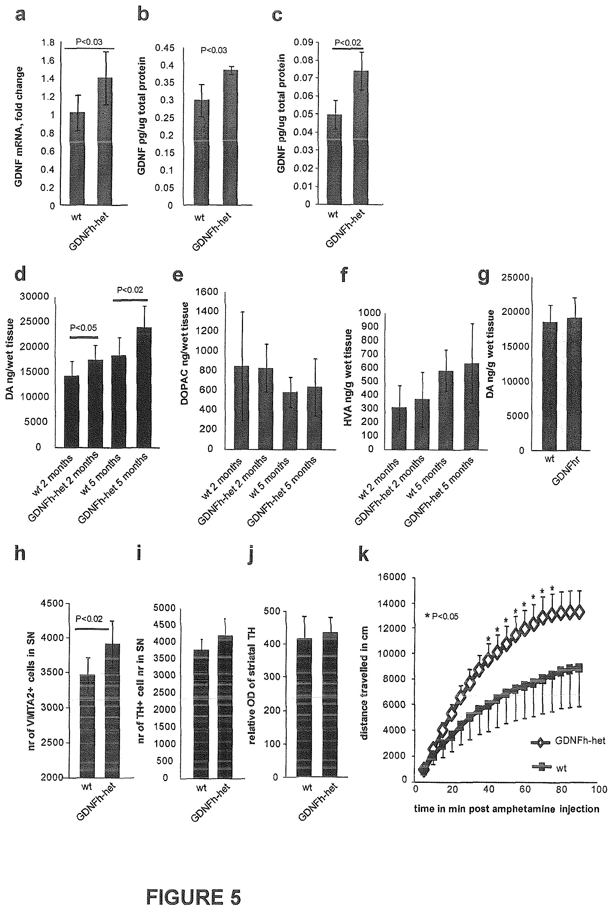

**Figure 5****.** Nigrostriatal dopamine system in adult heterozygous GDNF-3'UTR-crKO (GDNFh-het) mice. **a**, QPCR and **b,c** ELISA analysis of dorsal striatum and testis revealed that GDNF mRNA and protein levels are elevated by about 30% in GDNFh-het mice. n= 4-8 animals analyzed per genotype, represented data is compiled from 2-4 experiments. Error bars indicate SD.
   **d, e,f,** HPLC analysis of dopamine and its metabolite homovanillic acid (HVA) and 3,4-dihydroxyphenylacetic acid (DOPAC) levels in dorsal striatum at 2 and 5 months of age. 5-8 animals were analyzed per genotype, error bars indicate SD. **g,** dopamine levels in GDNFhr are not elevated at 3 months of age. **h**, Number of dopaminergic marker VMAT2 positive neurons in substantia nigra (SN) is increased in adult GDNFh-het animals by about 10-15%, **i,** Number of TH positive neurons in substantia nigra (SN) is increased in GDNFh-het animals, however, the increase is not statistically significant. **j**, optical density (OD) of TH striatal immunostaining reflecting TH levels and density of striatal dopaminergic innervation is unchanged in adult GDNFh-het mice. n=7 animals analyzed per genotype, cell numbers and OD of TH were analyzed by a blinded observer. **k**, Amphetamine (1mg/kg) induces increased spontaneous locomotor activity in GDNFh-het mice, suggesting enhanced dopaminergic transmission, n=9-10 animals analyzed per genotype. Experiment was repeated 3 times with similar results, representative experiment is shown. Error bars represent SD in all experiments.

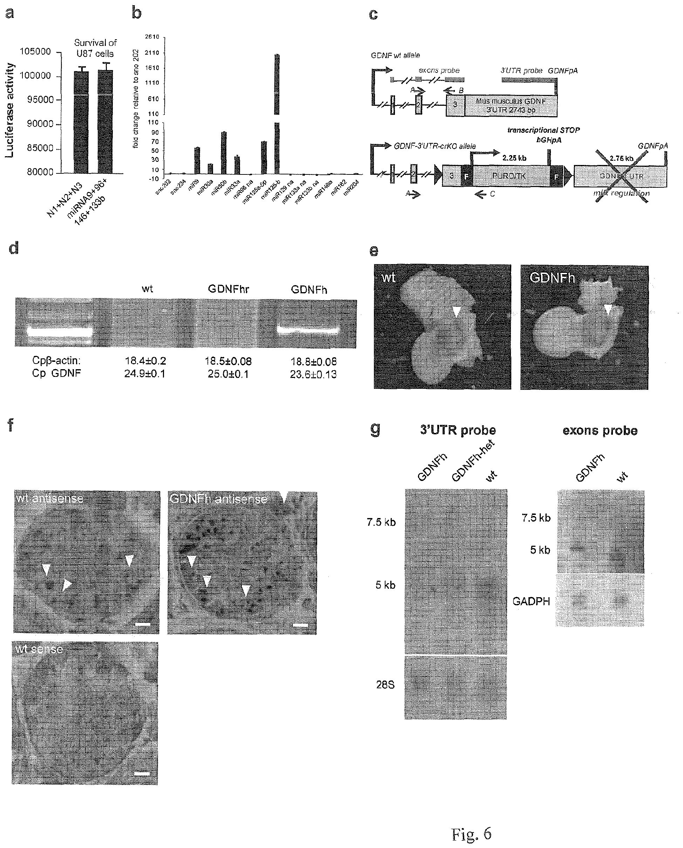

**Figure 6****.** Effects of miR overexpression on cellular survival, measurements of GDNF regulating miR levels in U87 cells and analysis of GDNF transcript length, sequence and expression site in GDNFh mice. **a**, Co-transfection of a combination of different pre-miR-s (Ambion) is not toxic to cells, as assayed with CellTiter-Glo Luminescent Cell Viability Assay (Promega), N-negative control. **b**, Levels of GDNF regulating mature miR-s relative to sno202 in astrocytoma cell line U87. Mature miR levels were assessed with TaqMan QPCR system (Applied Biosystems), 2 experiments were performed with 4 repeats per miR per QPCR run with similar results. Shown are results of a representative experiment, error bars represent SD. na-not applicable (Cp>35), indicating lack of expression of the given miR. **c,** targeting strategy of GDNF 3'UTR and exons (red) and 3'UTR (blue) probes used in in situ and Northern blot analysis. A and C indicate primers used in QPCR analysis of GDNF mRNA levels, primers A and C reflect primers used on **d,** for sequence analysis. **d**, The same cDNA used for QPCR analysis of GDNF mRNA levels in E18.5 testis (Fig. 3a) using primers A and B was used as a template for PCR reaction with primers A and C (**d**), the observed product in GDNFh mice was of expected length and was sequenced for further validation (data not shown). Cp values from QPCR to indicate cDNA quality are indicated below the lanes. **e**, In situ analysis of GDNF site of expression in GDNFh mice at the anatomical level in whole mount preparation of E11.5 hind limb. Establishment of correct limb innervation is known to involve precise site and timing of GDNF expression¹⁴. In GDNFh mice, the GDNF expression island (stained blue, indicated with white arrow heads) is indistinguishable from the wt, n=3 animals analyzed, shown are representative images, probe: GDNF exons. **f**, In situ analysis of GDNF site of expression in GDNFh mice at the cellular level in 5 µm thick paraffin slice from P7.5 seminiferous tubule. GDNF mRNA is mostly expressed by big cells, believed to be Sertoli cells² aligned to the periphery of the tubule (white arrow heads). GDNF expression site was very similar between genotypes, GDNF mRNA signal appeared stronger in GDNFh mice, overlapping results from the QPCR analysis (Fig. 3a). Scale bar 5µm. **g**, Northern blot analyses of GDNF mRNA from developing kidney at E18.5 when GDNF mRNA is particularly abundant significantly facilitating the analysis. GDNF exons probe (right panel) hybridizes to about 500 bp shorter transcript in GDNFh mice, matching the prediction that GDNF transcript from recombinant 3'UTR in the 3'UTR crKO mice is 500 bp shorter than the wt transcript (Fig. 6c, Fig. 2d). GDNF exons containing mRNA in GDNFh mice is also about 4-6 fold more abundant than in the wt (QPCR analysis of GDNF exons containing mRNA levels matching the depicted Northern blot data, alongside with an assessment of GDNF levels role in urogenital tract developed is in a greater detail addressed in manuscript submitted elsewhere). GDNF 3'UTR probe (left panel), hybridizes weakly to about 7.5 kb band likely reflecting readthrough from the bGHpA signal in GDNFh mice. Comparsion of left and right panel suggests that about 5 to maybe 10% of GDNF mRNA in GDNFh animals contains wt 3'UTR 3' to PURO/TK cassette. Such a transcript could in theory be regulated by miR-s. However, as shown on Fig. 2e and f, neither readthrough or suppression by miR-s was detected when such recombinant 3'UTR (rec-GDNF-3'UTR-crKO) was analysed in the reporter system in HEK 293 cells. This may reflect the effect of repositioning the miR binding sites away from their native loci within the 3'UTR, what may suppress the miR inhibition¹. The above readthrough also explains close to the detection limit signal occasionally observed in some, but not all in situ analyses of embryonal urogenital tract using GDNF 3'UTR probe (Fig. 3e and data not shown).
**Figure 7****.** Analysis of urogenital tract and its function in GDNFh and GDNFh-het mice. **a**, representative images of kidneys of GDNFh-het (left panel) and GDNFh (right panel) animals. The size of kidneys in GDNFh-het mice was slightly reduced at P7.5, but appeared histologically normal (data not shown, manuscript submitted elsewhere), whereas kidneys in GDNFh animals were tiny and functioned poorly (b, c), explaining their early death (Table 1). **b** and **c**, measurements of serum urea and creatinine levels reflecting kidney function at P7.5 demonstrated kidney failure in GDNFh animals. However, we found that serum urea levels are somewhat increased also in GDNFh-het animals. To study whether there is a correlation between serum urea and rostral brain dopamine levels we performed correlation analyses in individual animals and found no correlation between these two parameters in GDNFh-het mice (Table 2). **d** and **e**, serum urea and creatinine levels analysed in adult 2 month old animals (n=19 wt and n= 27 GDNFh-het animals were analyzed for urea and n=11 wt and n=17 GDNFh-het animals were analyzed for creatinine). **f**, bodyweight of adult GDNFh-het animals is normal (n=9 wt males, n=9 wt females, n=8 GDNFh-het males, n=10 GDNFh-het females). Error bars on all figures represent SD.

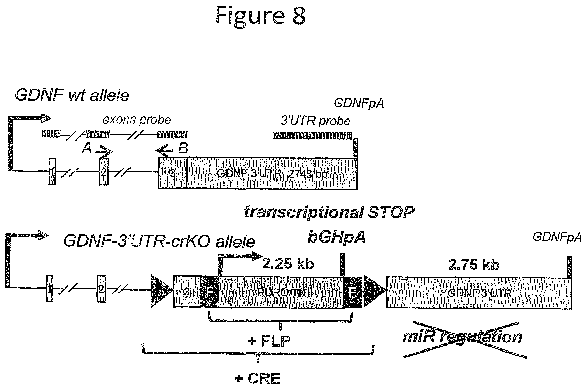

**Figure 8****.** GDNF 3'UTR is deleted by homologues recombination. Generation of 3'UTR conditionally reversible KO mice using the Flex system. Both GDNF wild type (GDNF wt) and functionally targeted GDNF (GDNF-3'UTR-crKO) alleles are shown. Depicted is a scheme for functionally targeting GDNF 3'UTR. A transcriptional stop and polyadenylation signal, such as bovine growth hormone polyadenylation signal bGHpA at the 3' of puro/TK casette functions as a transcriptional stop and ployadenylation signal for GDNF transcript. The direction of bGHpA cassette (red) can be reversed by Cre recombinase.

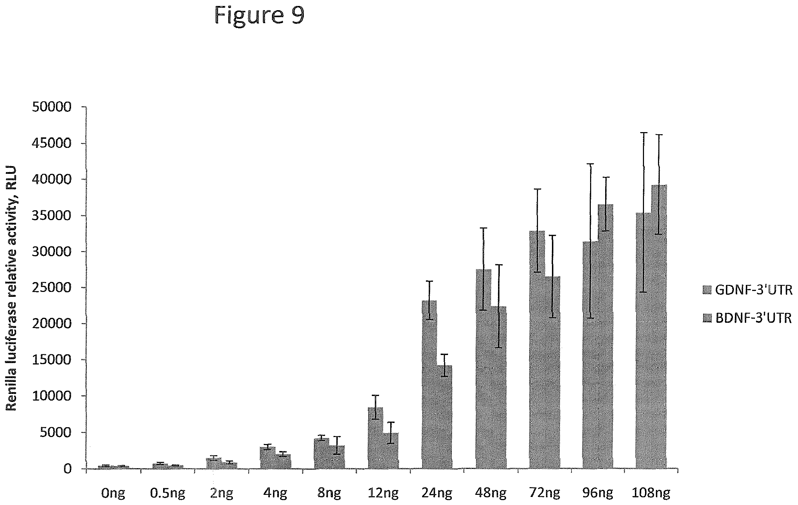

**Figure 9****.** Over-expression of GDNF, but not BDNF 3'UTR in U87 cells elevates Renilla-luciferase protein levels encoded by plasmid carrying GDNF 3'UTR * p<0.05, * * p<0.01. Renilla -GDNF-3'UTR plasmid was co-transfected to U87 cells from the left with 107.5, 106, 104, 100, 96, 84, 60, 36, 12 or 0 ng of either Firefly-GDNF3'UTR or Firefly-BDNF 3'UTR.

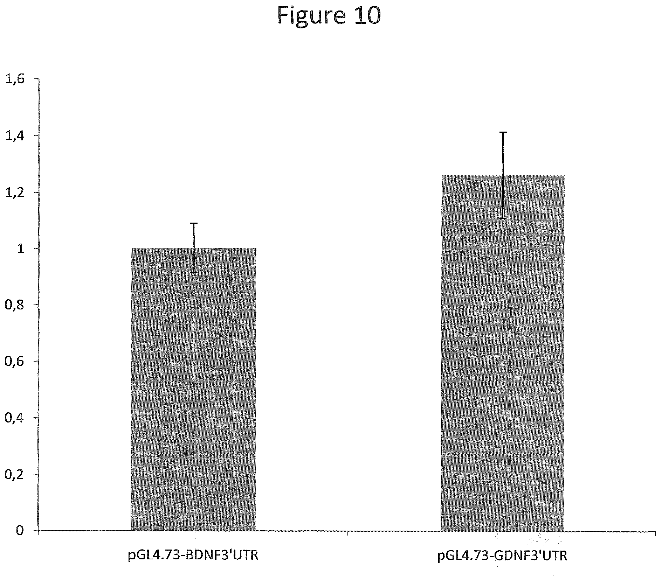

**Figure 10****.** Over-expression of GDNF, but not BDNF 3'UTR in U87 cells elevates endogenous GDNF mRNA levels, p<0.001.

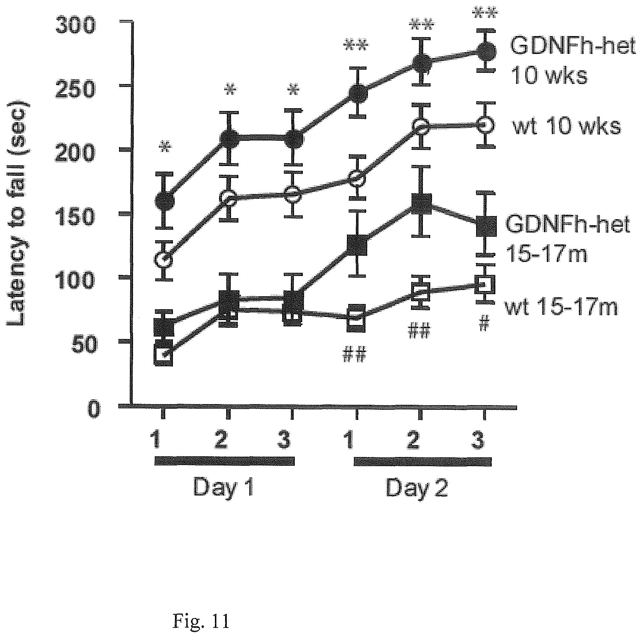

**Figure 11****.** Young mice perform better in Rotarod test. Accelerating Rotarod-measures coordination and motor learning-features severely affected in Parkinson's disease. 2 Days: 3 trials/day, Cut off 6 min (360 s). GDNFh-het males performed better in accelerating Rotarod stayed longer on the rod and had better balance.

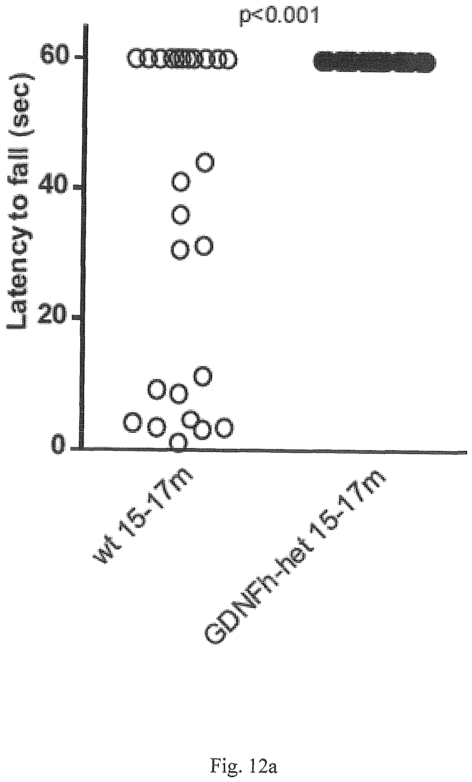

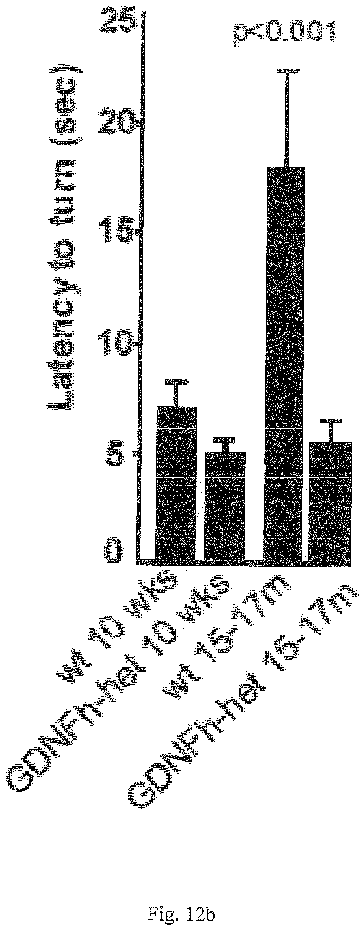

**Figure 12****. A,** vertical grid test,normal age-associated decline in the ability to position face upwards is fully rescued in GDNFh-het mice at 15-17 months of age. **B,** vertical grid test, latency to fall when positioned 33 head downwards is entirely rescued in 15-17m old GDNFh-het mice.

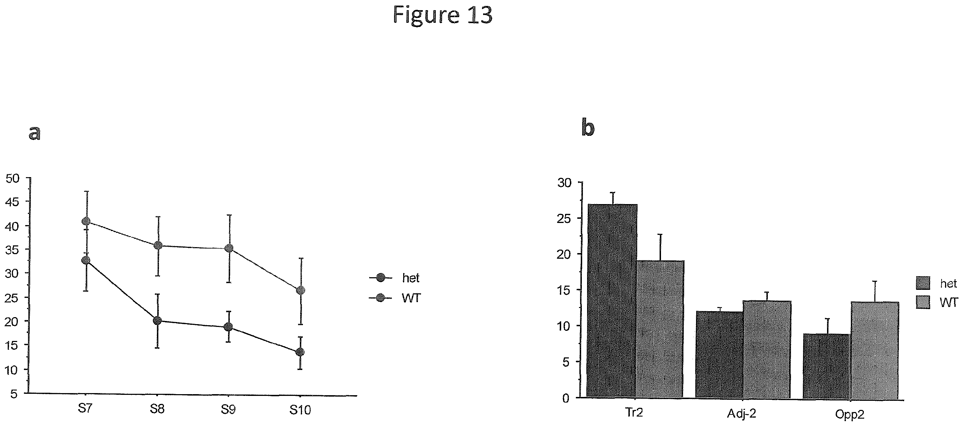

**Figure 13****.** Old GDNF hypermorphic mice also exhibit better learning and memory than wt controls. **a**, Reversal Learning: Escape Latency. **b**, Probe Trial 2: Time in Quadrants. Only HET mice show significant preference to the new platform location (* - p<0.05 compared to the time in target quadrant).

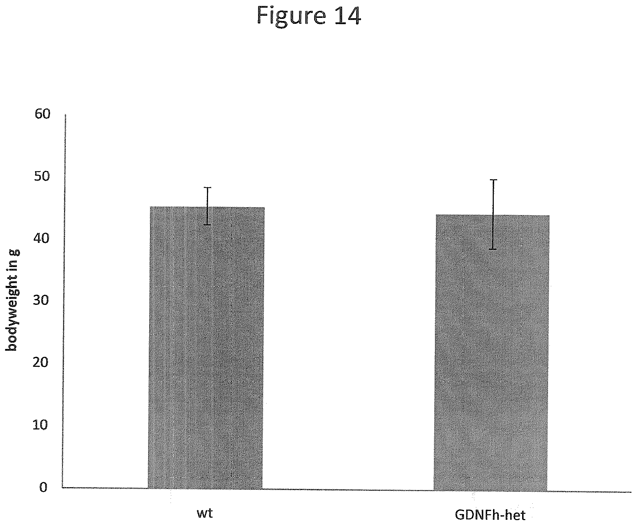

**Figure 14****.** Healthy aging is not due to reduced bodyweight in GDNF-3'UTR-crKO mice. n=6 GDNFh-het and n=7 wt male littermate control animals analyzed. Shown is the bodyweight at 17 months of age.

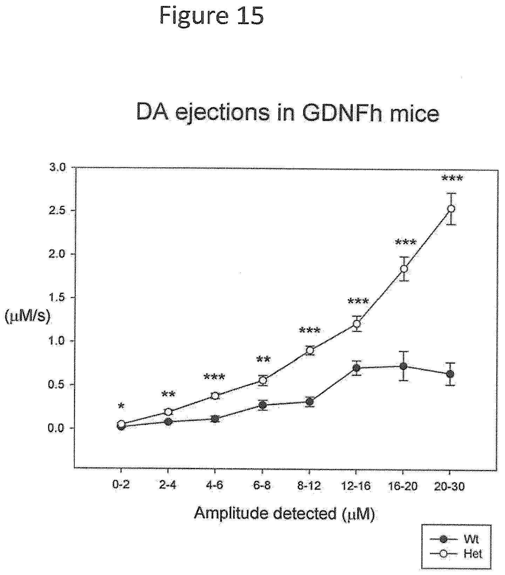

**Figure 15****.** Ampetometry with intrastriatal dopamine injections reveals elevated dopamine uptake in the striatum. *In vivo* carbon fibre chronoamperometry shows increased dopamine transporter (DAT) activity in striatum of GDNFh wild-type (Wt, N=4) over GDNFh heterozygote (Het, N=4) mice (one-way ANOVA, F=47,931, df=1, p<0,001) when dopamine (DA) is applied exogenously (200mM concentration). Dopamine peaks (mM) are separated into amplitude bins and plotted against uptake rate (mM /s; calculated using Michaelis-Menten first-order rate constant, k1). Post-hoc analysis shows significant differences at each level of amplitude (*=0,05; **=0,01; ***=0,001). Points denote mean values; error bars denote standard error (SEM). Pressure ejection of DA (200mM) in volumes of 10-475nl into striata of GDNFh wild-type (Wt) and heterozygote (Het) transgenic mice was employed to produce a range of amplitudes at each stereotaxic co-ordinate (AP +0,3mm; +1,0mm; ML ±1,8mm, DV-2,0; -2,5; -3,0; -3,5); data points are pooled for analysis.

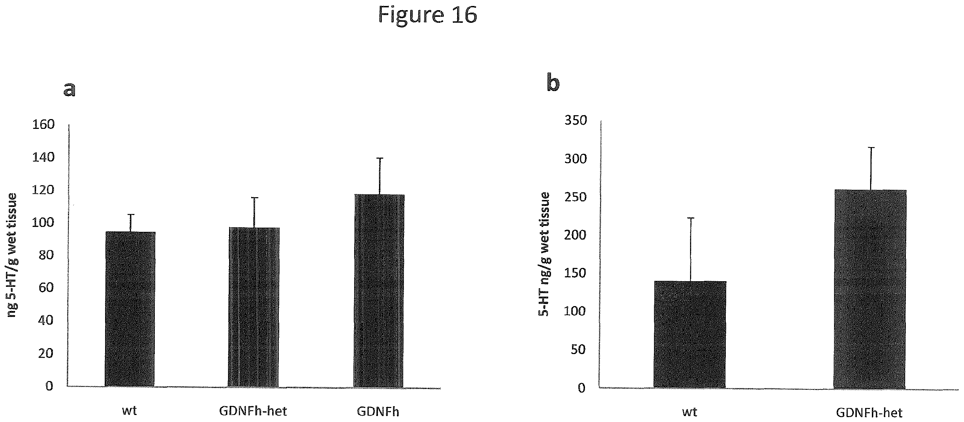

**Figure 16****.** GDNF levels also control brain serotonin levels in GDNF-3'UTR-crKO mice. **a**, 5-HT levels at P7 rostral brain, P<0,03. **b,** Dorsal striatal 5-HT level, ng/g wet tissue at 10 weeks of age, P<0,06.

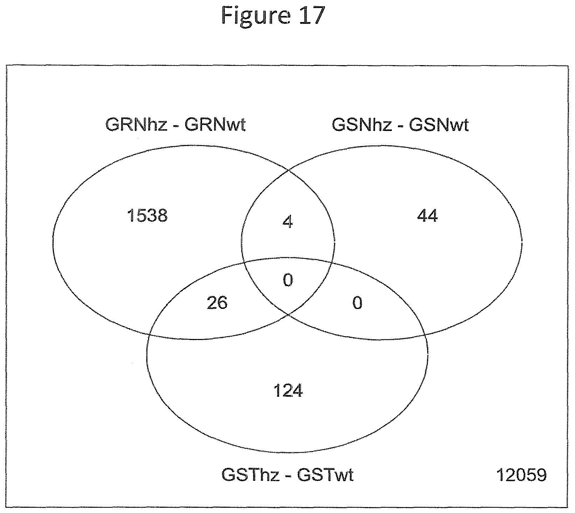

**Figure 17****.** Whole mouse Genome 4x 44K (G4122F) Oligo array. 3+3+3 wt v 3+3+3 GDNFh-het dorsal striatal (GSThz) and GDNFh-het SNpc (GSNhz) and GDNFh-het dorsal Raphe (GRNhz) RNA pools, cut off at P<0.01. There exist 124 changes in the dorsal striatum, 44 in SNpc, 1538 in dorsal Raphe nucleus, p<0.01.

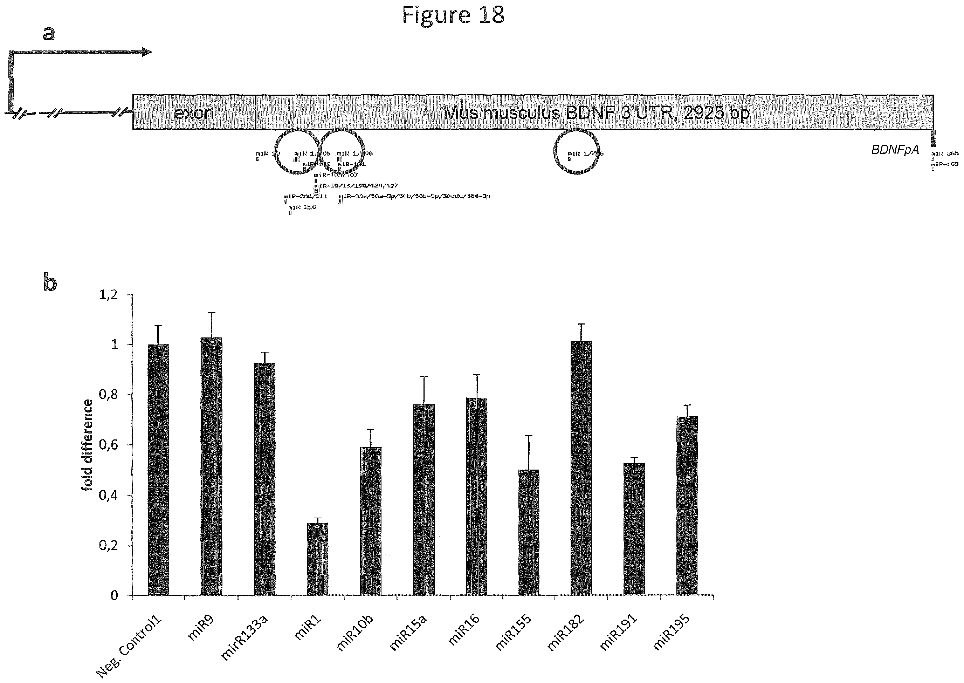

**Figure 18****.** miR regulation of BDNF 3'UTR in vitro. **a**, Shown are miR binding sites broadly conserved among vertebrates; Source: Blast, TragetScan. **b**, BDNF 3'UTR was cloned into Dual-Luciferase Reporter Assay System and co-transfected into HEK-293 cells either with negative control pre-miR N1 or putative BDNF 3'UTR regulating pre-miR-s at 10nM final concentration, as indicated.

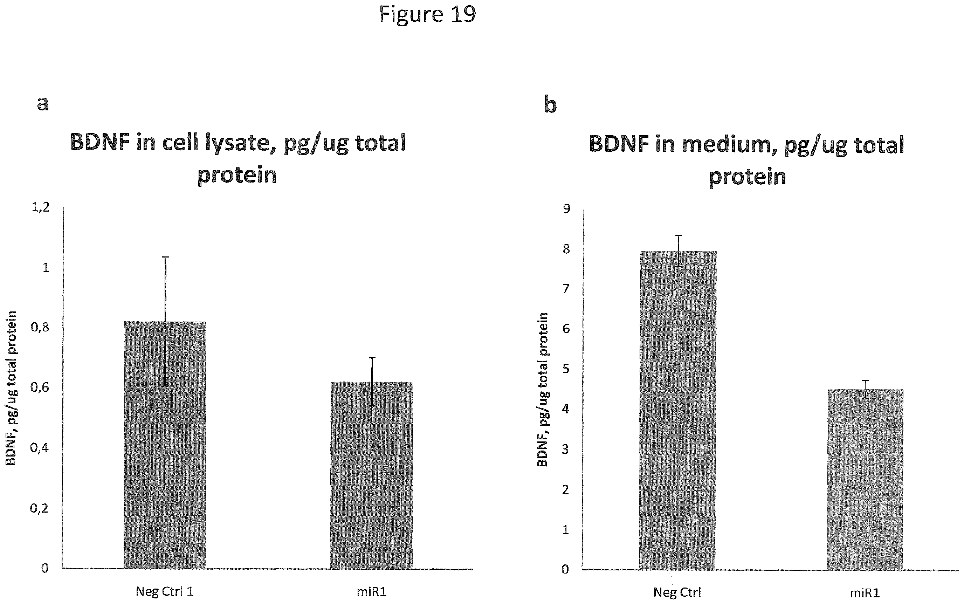

**Figure 19****.** miR1 suppresses BDNF levels in ARPE cells by 2 fold.

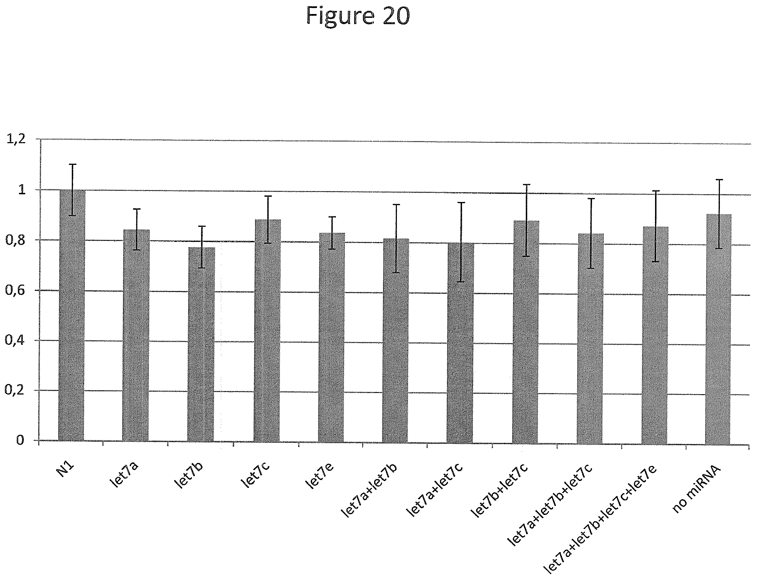

**Figure 20****.** Shown are the results of Renilla-NGF 3'UTR Luciferase assay (2 experiments, 10mM final miR concentration).

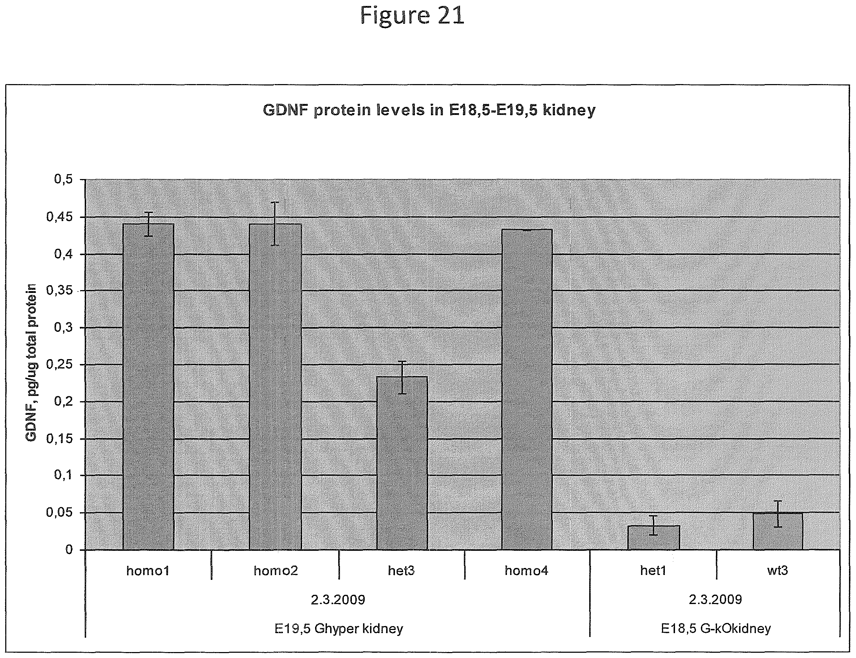

**Figure 21****.** GDNF protein levels at E18 in GDNF-3'UTR-cKO animal kidneys are elevated nearly 10 fold. Abbreviations: homo 1 = GDNF-3'UTR-cKO homozygous kidney; het3 = GDNF-3'UTR-cKO heterozygous kidney; het1 = GDNF knock-out heterozygous kidney.

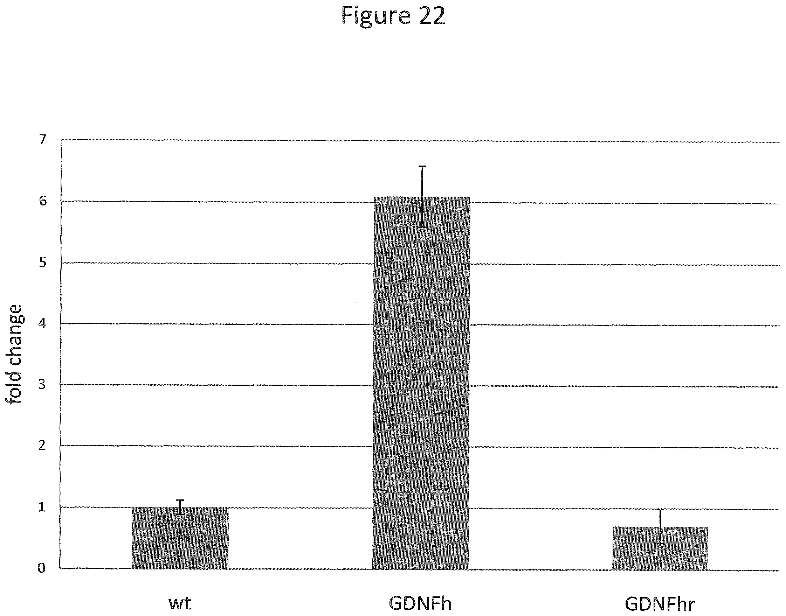

**Figure 22****.** mRNA levels of GDNF exons at E18,5 in GDNF-3'UTR-cKO animal kidneys are elevated 6-7 fold.

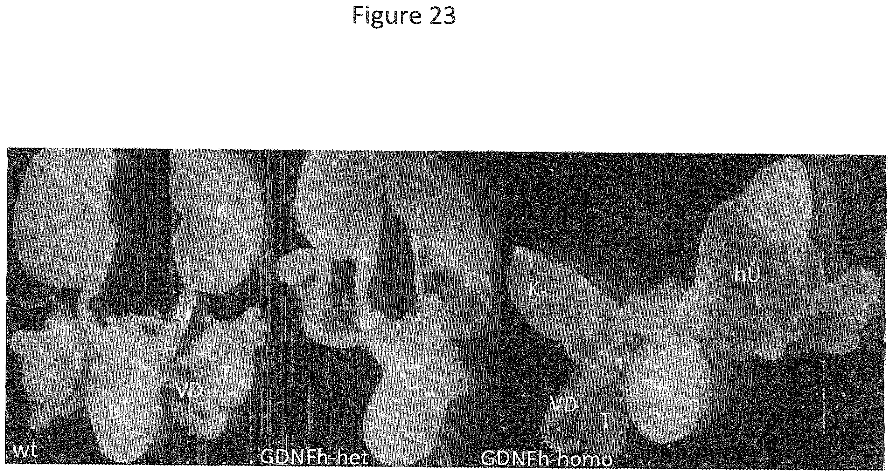

**Figure 23****.** GDNFh mice display CAKUT (congenital abnormality of kidney and urogenital tract) phenotype at P7. Abbreviations: K-kidney, U-ureter, hU-hydroureter, VD-vas deference, T-testis, B-bladder. Shown are reduced kidney size and unilateral mild hyrdourter in GDNFh heterozygous (GDNFh-het) mice and severely reduced kidney size, hydroureter, severely shortened ureters, blood filled testis likely resulting from misconnected vas deference to ureter or urogenital sinus causing the flow of urine into the testis, indication of multiple cystic ureters (left kidney) in the GDNFh homozygous (GDNFh-homo) mice. GDNF-het UGT is from a female mouse.

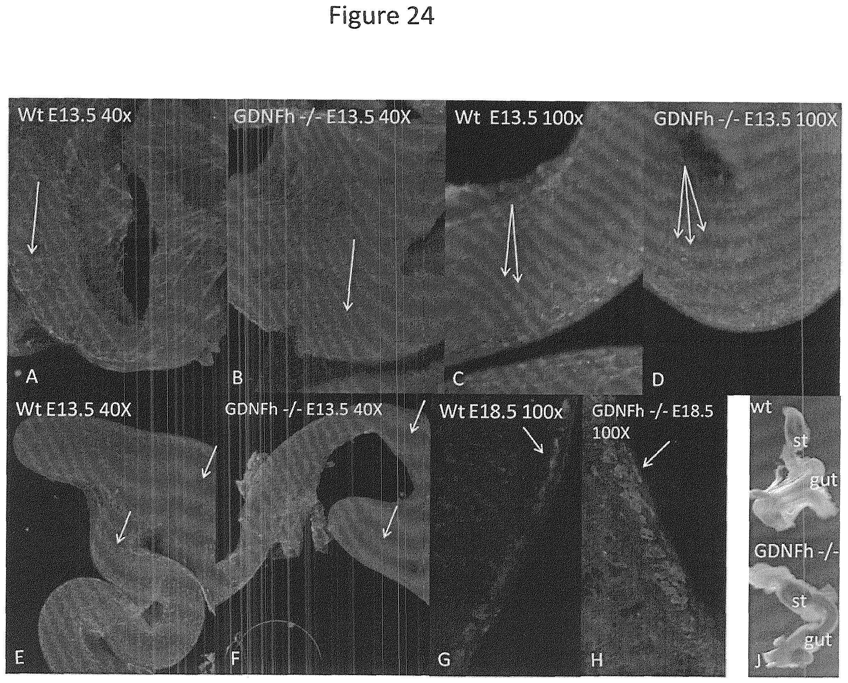

**Figure 24****.** Mice with elevated endogenous GDNF in the gut display hypergangliosis of the gut. miRs regulate GDNF and consequently enteric nerve cell number, migration and differentiation in the developing GI tract. Note the lack of mature fibers and increased number of ENCC cells bodies as visualized with pan-neuronal marker PGP9.5 immunostaining at E13.5 wholemount preparation of the GI tract in the stomach (arrows on A,B). C,D illustrate the elevated ENCC number in the duodenum at E13.5 in the same preparation, E,F show the lack of ENCC cells in the caecum in the same preparation, G,H show that in the GDNFh -/- mice distal gut hypogangliosis (E,F) is changed to hypergangliosis by E18.5 using PGP 9.5 immunostaining of 4µm paraffin sections from the colon J is whole mount in situ analysis of GDNF mRNA (blue) in the GI tract at E11, darker blue reflects elevated GDNF mRNA levels.

### DETAILED DESCRIPTION OF THE INVENTION

Currently, the function of a gene *in vivo* is studied by either overexpressing it using a transgenic approach, or by knocking it out. The main problem associated with transgenic overexpression using either cDNA or bacterial artificial chromosome based strategies is misexpression in space and time. Moreover, often a gene has structural and/or functional homologues in which case the KO animal may lack phenotype, giving a false negative result. Therefore, a method that would enable to overexpress a gene product in the natively expressing cells only would benefit those fields of biology where genetically modified animal models are used. This is especially important in studying processes where temporally and spatially tightly controlled protein gradients determine the phenotype. Such processes are common i.e. during the development, but they are especially relevant in the maturation and maintenance of the brain. There, the gradients of target-derived neurotrophic factors and other guidance-cue molecules determine which neuronal contacts come to exist and are maintained. Since the precise arrangement of neuronal contacts is believed to underlie brain function, a knowledge on target derived NTFs and their effects would be important for understanding how the brain develops and functions, and for designing drugs to treat its disorders.

Recently, it has been experimentally shown that micro RNA-s (miRs), about 20-22 bp single stranded RNA molecules, control the levels of hundreds of mammalian gene products by binding to the 3' UTRs of their target mRNA-s, effectively destabilizing them and/or suppressing their translation. Moreover, bioinformatics approaches predict that the levels of more than half of mammalian genes are controlled by miR-s¹. However, most often a 3'UTR is regulated by a combination of multiple miR-s, and a single miR is predicted to regulate over 100 mRNA-s, making it difficult to analyze the biological importance of miR regulation of a given gene product, particularly, in vivo.

Towards that end, we studied GDNF, a NTF that promotes axonal branching and survival of midbrain dopamine (DA) neurons what specifically degenerate in currently incurable Parkinson's disease. GDNF and it close relative NRTN are clinically relevant and have been, and are at present, tested in clinical trials with highly promising, but yet with somewhat conflicting outcomes highlighting a need for the better understanding of their biology in vivo and improved treatment 10⁻¹² Moreover, current knowledge on the role of endogenous GDNF in brain DA system development and function is poor, since "classical" GDNF coding region KO mice have intact DA system but die at birth due to the lack of kidneys, whereas the brain DA system maturation is largely post-natal. How GDNF levels are regulated in vivo is largely unknown.

Here we show that i) GDNF levels are regulated via its 3'UTR by different miR-s in vitro ii) knocking out of GDNF 3'UTR in vivo results in overexpression of GDNF in GDNF naturally expressing cells iii) GDNF levels regulate postnatal development and function of the brain DA system.

Here we show that GDNF levels are regulated by miR-s via its 3'UTR in vitro and that knocking out the 3'UTR of GDNF in vivo leads to up to 10 fold elevation of endogenous GDNF levels in different tissues in GDNF natively expressing cells. The resulting GDNF hypermorphic animals display elevated brain DA levels and DA neuron number demonstrating for the first time that endogenous GDNF acts as a target derived neurotrophic factor fine tuning the function and cell number of the postnatal DA system. These results reinforce the potential use of GDNF as a drug for the treatment of PD. Notably, potential adverse effects of exogenous GDNF overexpression, such as adult TH protein levels downregulation observed in animals overexpressing GDNF from lentiviral vectors in the striatum¹¹⁻¹², were lacking. Unlike in the aforementioned cases were despite of the exogenous GDNF overexpression ranging from several tens to hundreds of folds striatal dopamine levels were not increased¹¹⁻¹², dopamine levels were clearly enhanced in GDNFh mice where GDNF levels were elevated only 2-5 fold at P7 and about 30% in adult mice. This result may suggest that the site of GDNF expression, rather than its amounts, may be critical and that even modest elevation of GDNF levels in the correct site, bona fide in the cells what naturally express GDNF, may be superior over robust GDNF over expression as has been attempted so far in animal studies and clinical trials. The latter has been at least in part due to the lack of knowledge on molecular mechanisms controlling the endogenous GDNF levels. Our results share light into mechanism of endogenous GDNF levels control and highlight GDNF 3'UTR and its regulating miR-s as a potential new drug target for the treatment of PD and potentially other neurodegenerative diseases. They also show that 3'UTR mediated levels control of a gene product can be physiologically important and that next to the classical KO method of the coding sequence, KO of the 3'UTR may be at least as informative. It should be noted however, that intracellular localization of about 15 mRNA-s can depend on signal sequences within their 3'UTR-s¹³. Alongside with studies analyzing the effects of endogenous GDNF levels on the structure and function of the brain dopamine system in a greater detail, we also aim to analyze this parameter for GDNF mRNA in the future. Finally, we would like to suggest that rescue experiments using crossings of the hypermorphic animals generated by knocking out the 3'UTR-s of miR regulated genes to mouse models of human diseases could potentially be useful for screening for novel drug targets.

We hypothesized, that knocking out the 3'UTR of a miR-controlled gene would result in overexpression of the gene product exclusively in those cells that already transcribe the gene. We studied GDNF and first showed that GDNF levels are controlled by multiple miR-s *in vitro.* Next, we generated mice in which the native GDNF 3'UTR is reversibly substituted with a sequence that is not regulated by miR-s. Such GDNF 3'UTR KO animals expressed up to 10 fold more GDNF in natively expressing cells. Compared to healthy littermate controls, GDNF 3'UTR KO mice displayed elevated brain dopamine levels, elevated number of dopaminergic neurons in substantia nigra and enhanced dopaminergic transmission as revealed by elevated amphetamine induced locomotor activity. It is important to note that GDNF has clinical potential because it has been, and currently is, in clinical trials for treating currently incurable PD were dopamine neurons specifically degenerate. The results from clinical trials are promising but so far variable highlighting a need for the better understanding of GDNF biology *in vivo.* The latter has been limited due to the fact that mice lacking GDNF gene die at birth due to the lack of kidneys, whereas brain dopamine system maturation is largely post natal. How endogenous GDNF levels are controlled has also remained poorly understood.

Our results share light into the mechanism of endogenous GDNF levels regulation and show that endogenous GDNF acts as post natal target derived neurotrophic factor for midbrain dopamine neurons. They also reinforce the potential of GDNF as potential drug to treat PD and suggest GDNF 3'UTR and its regulating miR-s as new drug target, potentially enabling to avoid some problems currently associated with striatal GDNF overexpression. Moreover, our results also suggest that next to the classical KO method of the coding region, KO of the 3'UTR of a miR regulated gene may be a new, informative approach to study gene function as it enables to overexpress a gene in those cells only what contain the transcript thereby avoiding the misexpression problem. Our work also highlights the physiological relevance of 3'UTRs suggesting that the 3'UTR KO method as suggested here may enable to ask and answer new types of biological questions and perform novel types of drug screens.

The present specification thus shows that the 3'UTR of glial cell-line derived neurotrophic factor (GDNF) is negatively regulated by multiple microRNAs and that *in vivo* replacement of GDNF 3'UTR with a recombinant 3'UTR, devoid of microRNA repression, results in GDNF overexpression in natively-expressing cells only. Compared to healthy littermate controls, young mice with elevated endogenous GDNF levels displayed enhanced dopaminergic function and improved motor coordination. Moreover, normal age-associated decline in motor performance, thought to result from a multifactorial process, was overcome by enhanced endogenous GDNF levels. This was achieved without side effects associated with pharmacological enhancement of the dopamine system or ectopic GDNF applications. Our findings illustrate the potential of replacing 3'UTRs *in vivo* as an alternative approach to study gene function and to reveal new drug targets.

Accordingly, the present specification provides a transgenic KO non-human animal comprising a heterozygous or homozygous deletion or functional deletion of the gene's native 3'UTR at least in one of its endogenous gene loci, wherein the disrupted endogenous gene is transcribed into an mRNA without its native 3'UTR. In other words, the disrupted endogenous gene is transcribed into an mRNA so that said mRNA carries at least partially modified 3'UTR instead of its native 3'UTR. Advantageously, said modified 3'UTR is devoid of microRNA binding sites.

Preferably, the KO animal comprises a deletion or functional deletion of 3'UTR in the gene GDNF, NGF or BDNF.

More preferably, the KO animal comprises a deletion or functional deletion of 3'UTR of GDNF encoding gene.

Preferably, said KO non-human animal is selected from the group consisting of a rodent, rabbit, sheep, pig, goat, and cattle.

The present specification is also directed to a KO vector construct containing a selectable marker gene and stretches of genomic DNA spanning the regions 5' and 3' to the 3'UTR of the gene of interest effective to remove said 3' UTR and substitute it with a recombinant 3'UTR, under conditions of homologous recombination, wherein said vector is suitable for producing native 3'UTR KO or functional KO non-human animals. The vector or the transgenic non-human animal comprising functional 3'UTR KO of GDNF gene preferably comprises the sequence of SEQ ID NO:1. The sequence according to SEQ ID NO:2 consisting additional flanking FRT sites enables production of also conditional or conditionally removable knockouts. Preferably, said vector construct is suitable for producing native 3'UTR KO or functional KO when the gene is GDNF, NGF or BDNF.

Most preferably, said vector construct is suitable for producing native 3'UTR KO or functional KO when the gene is GDNF.

The present specification is also related to a method for producing a KO non-human animal, the method comprising a step of introducing the vector suitable for producing native 3'UTR KO or functional KO non-human animals into embryonic stem cells of a non-human animal.

The present specification is also directed to a method for producing homozygous or heterozygous 3'UTR KO non-human animal, the method comprising a step of mating together a male and a female animal each heterozygous or one wild type for said disrupted gene and selecting progeny that are homozygous or heterozygous for said disrupted 3'UTR of a gene.

The present specification also encompasses a progeny of said transgenic KO non-human animal, obtained by breeding it with the same or any other genotype.

The present specification is also related to a transgenic KO non-human animal, which is a mouse.

The present specification is also directed to a cell line of said transgenic KO non-human animals.

Preferably the cell line is a murine cell line.

In one case, the present specification includes a use of said transgenic 3'UTR KO non-human animals as models for examination of behavior during the development of a neurodegenerative disease, or said cell lines for examination of pathobiochemical, immunobiological, neurological as well as histochemical effects of neurodegenerative diseases, physiological and molecular biological correlation of the disease, for evaluation of potentially useful compounds for treating and/or preventing a disease, for studies of drug effects, and for determination of effective drug doses and toxicity.

In another case, the present specification also includes a use of said transgenic 3'UTR KO non-human animals as models for identifying proteins and/or 3'UTRs and 3'UTR regulating molecules such as micro RNA-s as drug targets for the treatment of human diseases including, but not limited to Parkinson's disease, Alzheimer's disease, Huntington's disease, dementia, depression, Schizophrenia, Amyotrophic Lateral Sclerosis (ALS), spinal cord injury, age associated memory decline, age related drop in physical activity, age related decline in motor coordination; preferably, for use in the treatment of Parkinson's disease or age related decline in motorcoordination.

In a preferred case, the present specification includes the use of said transgenic 3'UTR KO non-human animals as models, wherein said neurodegenerative disease is Parkinson's disease, Alzheimer's disease, Huntington's disease, dementia, Amyotrophic Lateral Sclerosis (ALS), spinal cord injury, age associated memory decline, age related drop in physical activity, age related decline in motor coordination.

The present specification also encompasses *in vitro* and *in vivo* methods for modulating the expression levels of GDNF, BDNF or NGF polypeptides in a non-human animal, and in human, the method comprising a step of modulating 3'UTR regulation of endogenous GDNF, BDNF or NGF mRNA with short interfering RNAs (siRNAs), double-stranded RNAs (dsRNAs), native and synthetic micro-RNAs (miRNAs), short hairpin RNAs (shRNAs), miRNA sponges, anti-miRNAs, morpholinos, miRNA target site protectors, or antisense oligonucleotides. In other words, the method is performed by introducing into cells, including those cells naturally expressing endogenous GDNF, BDNF or NGF mRNA short interfering RNAs (siRNAs), double-stranded RNAs (dsRNAs), native and synthetic micro-RNAs (miRNAs), short hairpin RNAs (shRNAs), miRNA sponges, anti-miRNAs, morpholinos, miRNA target site protectors, or antisense oligonucleotides modulating 3'UTR regulation of said endogenous GDNF, BDNF or NGF mRNA.

Preferably, the expression level of GDNF gene is modulated in which case the target micro RNAs to be regulated are selected from the group consisting of miR-133a, miR-133b, miR-125a-5p, miR-125b-5p, miR-30a, miR-30b, miR-96, miR-9, and miR-146 (see Example 2 and Fig. 2).

Preferably, the expression level of BDNF gene is modulated and target micro RNAs to be regulated are selected from the group consisting of miR-1, miR-10b, miR15a, miR16, miR-155, miR-182, miR-191, and miR-195 (see Fig. 18).

Preferably, the expression level of NGF gene is modulated and target micro RNAs to be regulated are selected from the group consisting of miR let-7a, miR let-7b, miR let-7c, and miR let-7e (see Fig. 20).

The present specification further encompasses *in vitro* and *in vivo* methods for modulating the expression levels of GDNF, BDNF or NGF polypeptides in a non-human animal, and in human, the method comprising a step of contacting a miRNA sponge containing at least part of, or full one copy of 3'UTR of endogenous GDNF, BDNF or NGF mRNA with miRNAs.

Aspects of the present specification are also *in vitro* and *in vivo* methods for modulating the expression levels of GDNF, BDNF or NGF polypeptides in a non-human animal, and in human, the method comprising a step of overexpressing GDNF, BDNF, or NGF 3'UTR, or parts of it in one or more copies leading to relief of suppression of endogenous GDNF, BDNF and NGF by endogenous inhibitors what act over the 3'UTR.

Another aspect of the specification is a short interfering RNA (siRNA), double-stranded RNA (dsRNA), native and synthetic micro-RNA (miRNA), short hairpin RNA (shRNA), miRNA sponge, anti-miRNA, morpholino, miRNA target site protector, or antisense oligonucleotide targeting miR-133a, miR-133b, miR-125a-5p, miR-125b-5p, miR-30a, miR-30b, miR-96, miR-9, or miR-146 and increasing the expression level of GDNF in a cell expressing endogenous GDNF, for use in the treatment of Parkinson's disease, Alzheimer's disease, Huntington's disease, dementia, ALS, spinal cord injury, age associated memory decline, age related drop in physical activity, or age related decline in motorcoordination; preferably, for use in the treatment of Parkinson's disease or age related decline in motorcoordination.

The present specification also provides a short interfering RNA (siRNA), double-stranded RNA (dsRNA), native and synthetic micro-RNA (miRNA), short hairpin RNA (shRNA), miRNA sponge, anti-miRNA, morpholino, miRNA target site protector, or antisense oligonucleotide targeting miR-1, miR-10b, miR15a, miR16, miR-155, miR-182, miR-191, and miR-195 and increasing the expression level of BDNF in a cell expressing endogenous BDNF, for use in the treatment of Parkinson's disease, Alzheimer's disease, Huntington's disease, dementia, ALS, spinal cord injury, age associated memory decline, age related drop in physical activity, or age related decline in motorcoordination; preferably, for use in the treatment of Alzheimer's disease and Parkinson's disease or age related decline in motorcoordination.

The present specification further provides a short interfering RNA (siRNA), double-stranded RNA (dsRNA), native and synthetic micro-RNA (miRNA), short hairpin RNA (shRNA), miRNA sponge, anti-miRNA, morpholino, miRNA target site protector, or antisense oligonucleotide targeting miR let-7a, miR let-7b, miR let-7c, and miR let-7e and increasing the expression level of NGF in a cell expressing endogenous NGF, for use in the treatment of chronical neuropathic pain, Parkinson's disease, Alzheimer's disease, Huntington's disease, dementia, ALS, spinal cord injury, age associated memory decline, age related drop in physical activity, or age related decline in motorcoordination; preferably, for use in the treatment of chronical neuropathic pain, Parkinson's disease or age related decline in motorcoordination.

The present invention is further described in the following examples, which are not intended to limit the scope of the invention.

### MATERIALS AND METHODS

Pre-miR-s were purchased from Ambion, PS bonded, LNA based anti-miR-s from Exiqon. miR levels were assessed using TaqMan QPCR kit (Applied Biosystems), miR effects on recombinant 3'UTR-s were assessed using Dual-Luciferase Reporter Assay System (Promega). GDNF protein levels in cell culture medium and testis lysate was analysed using GDNF ELISA (Promega) and in the brain using GDNF ELISA from RnD. RNA was isolated using TRI Reagent (Molecular Research Center, USA) DNAse (Promega) treated, reverse transcription reaction was performed with RevertAid reverse transcriptase (Fermentas). QPCR analysis was done using LightCycler^{®} 480 Real-Time PCR System (Roche). For Northen blotting poly A enrichment was carried out using NucleoTrap mRNA kit. Specific RNA-s were detected using probes as indicated above with DIG based nucleotide detection system (Roche Applied Science). GDNF-3'UTR-crKO mice were generated using standard procedures and housed and studied according to the legislation in Finland. Serum urea and creatinine were measured with standard kits (BioAssay Sytems). Brain immunohistochemistry and HPLC detection of dopamine and its metabolites were detected in essence as in ¹⁵. Amphetamine (1mg/kg) induced locomotor activity was measured in open-field activity monitors; MED Associates, St. Albans, GA.

### EXAMPLE 1. GDNF family and putative miR regulation

GDNF and its family members NRTN, ARTN, PSPN are NTFs involved in diverse biological processes including development of kidneys, enteric neurons, sub-populations of sympathetic and GABA-gic neurons. They signal by first binding, with some degree of cross-reactivity, to their primary receptors GFRal-4 respectively, followed by dimerization and autophosphorylation of the signaling component of the receptor complex, RET. Due to their clinical potential, we were interested in the mechanisms what control the levels of endogenous GDNF family members and their receptors. Using the currently available bioinformatics tools¹ we analyzed the 3'UTRs of GDNF, NRTN, ARTN, PSPN, GFRal-4 and RET and found that the 3'UTR-s of only GDNF and RET contain broadly conserved seed sequences for multiple miR families and general sequence conservation (Fig. 1a and data not shown). Next, we asked, whether the bioinformatics predicted GDNF regulating miR-s are expressed in tissues where GDNF levels have been shown to be important during the development. Those include developing testis where GDNF levels control cell fate decisions of the stem cells for spermatogenesis², developing rostral brain where GDNF levels regulate migration and maturation of cortical GABAegic neurons and olfactory bulb interneurons³⁻⁴ at postnatal day 7 (P7) and dorsal striatum from adult (10 week old) mice, where GDNF levels may contribute to maintenance of dopaminergic fibers specifically lost in Parkinson's disease in aging mice and man⁵. We found that most predicted miR-s were expressed but levels varied considerably between different tissues (Fig. 1b).

### EXAMPLE 2. GDNF levels are regulated via its 3'UTR by multiple miR-s in vitro

Next, we asked, whether GDNF 3'UTR can be specifically regulated by the predicted miR-s. Because the number of bioinformatics predicted putative GDNF regulating miRs varies substantially depending on the search engine and stringency conditions, we chose nine miR-s for further analysis based on known expression profiles⁶, Fig. 1b, and length and evolutionary conservation of the seed sequence and, in case of miR-133-s, the presumed involvement in Parkinson's disease^{1,7}. We tested GDNF full-length 3'UTR in the Dual-Glo reporter system (ref) in the presence of different control miR-s (N) and putative GDNF regulating miR-133a, miR-133b, miR-125a-5p, miR-125b-5p, miR-30a, miR-30b, miR-9, miR-96 and miR-146. We found that all the above miR-s, each to a different extent, negatively regulated expression from reporter construct containing GDNF 3'UTR independent whether GDNF 3'UTR was cloned after luciferase derived from sea pansy (Renilla reniformis) or Photinini firefly (Photinus pyralis) at 1, 10 and 100 nM final miR concentrations (Fig. 2a, b and c and data not shown). Next, we asked, whether endogenous GDNF levels can be regulated with miR-s. Towards that end we used human astrocytoma cell line U87, which is one of the few, if not the only cell line what produces and secretes into the culture medium enough GDNF derived from GDNF's endogenous locus detectable with currently available methods⁸. We hypothesized, that different miR-s regulate GDNF levels in an additive manner, as has been shown for several transcripts derived from other genes¹. We found that miR-s in combination suppress endogenous GDNF levels more efficiently than each alone (Fig. 2g) without affecting cellular survival (Fig. 6a). Although numbers vary for each locus, miR suppressive effect on a single gene product levels is thought to be on average about 80% mediated via destabilization of the transcript and about 20% by translational suppression¹. Analysis of GDNF mRNA levels in U87 cells after transfection of four different control,- or four GDNF protein levels most effectively suppressing miR-s showed that endogenous GDNF mRNA levels are reduced by about 40% by this miR combination (Fig. 2i). Next we asked, whether inhibition of endogenous miR-s results in elevated GDNF mRNA levels. First, we asked what GDNF levels regulating miR-s are expressed in U87 cells and found that compared to the other GDNF regulating miR-s (Fig. 2a), miR-125b-5p, 125a-5p, miR30b and miR9 are expressed relatively abundantly in U87 cells (Fig. 6b). As shown on Fig. 2h, anti-miR based inhibition of the above four miR-s resulted in about 30% upregulation of endogenous GDNF mRNA levels in those cells.

### EXAMPLE 3. Generation of GDNF 3'UTR conditionally reversible KO (GDNF-3'UTR-crKO) mice

Next, we wanted to know the *in vivo* significance of GDNF levels regulation via its 3'UTR. However, it is technically challenging, if not impossible to specifically knock out all GDNF regulating miR genes or mutate their putative binding sites in GDNF 3'UTR. Moreover, our data suggests that GDNF 3'UTR is regulated by a combination of multiple miR-s, where deletion of a single miR gene may have no,- or very little effect. Finally, since single miR is predicted to regulate on average about 200 different mRNA-s, knocking out one miR also likely affects other targets mRNA-s ^{1,9}. Therefore, we decided to take a novel approach and reversibly knock-out the 3'UTR of GDNF by substituting its ca 2.75 kb miR regulated 3'UTR with a cassette of a comparable length (2.25 kb) lacking the binding sites for GDNF regulating miR-s, containing a strong mammalian transcriptional stop signal (bovine growth hormone polyadenylation signal, bGHpA) and flanked with FRT sequences for reversal of the targeted allele to wt with FLP recombinase (Fig. 2d). We called such an allele GDNF 3'UTR conditionally reversible knock out allele (GDNF-3'UTR-crKO). Before homologous recombination in the mouse embryonic stem cells, we analyzed the obtained recombinant GDNF 3'UTR (rec GDNF-3'UTR-crKO, Fig. 2d) in a reporter construct 3' to luciferase coding sequence from sea pansy and found that GDNF native 3'UTR is no longer transcribed from such a construct (Fig. 2e) and that GDNF regulating miR -s are no longer able to inhibit expression from such a construct (Fig. 2f).

### EXAMPLE 4. GDNF expression in GDNF-3'UTR-crKO animals

After generating the GDNF-3'UTR-crKO animals using routine methods, we first analyzed GDNF levels and site of expression. For this purpose, we chose organs and developmental times where GDNF mRNA and/or protein levels are known to be at the highest levels and thus readily detectable with currently available methods (refs for GDNF expression levels, esp. in developing kidney and testis). We found that at E18,5 in the testis, GDNF mRNA levels are elevated 2,5 fold in the heterozygous,- and ca 5 fold in GDNF-3'UTR-crKO homozygous mice. For the reasons of simplicity, homozygous animals were designated GDNF hypermorphs (GDNFh) and heterozygous animals as GDNFh-het mice. Restoration of transcription to wt GDNF 3'UTR by crosses to Deleter-FLP line (Fig. 2d) returned GDNF mRNA levels to the wt level and the allele was designated as GDNFh-rescued (GDNFhr) allele (Fig. 3a). GDNF protein levels were similarly elevated in E18,5 testis (Fig. 3b). During our initial analysis of the rostral brain at P7 we found that compared to E18,5 testis were GDNF mRNA levels were elevated ca 5 fold, GDNF mRNA was "only" about 70% upregulated in GDNFh rostral brain. To assess sensitivity of our QPCR analysis using primers A and B as indicated on Fig. 2d, we included heterozygous GDNF coding region knock out (GDNF-KO-het) animals to our analysis obtained by crossing the GDNFhr animals to Deleter-Cre line (Fig. 2d). Using cDNA-s derived from the above genotypes in QPCR analysis we confirmed that in P7 rostral brain, GDNF mRNA levels were elevated about 30% and 70% in GDNF-het and GDNFh animals respectively (Fig. 3c). GDNF protein levels in P7 rostral brain on the other hand were elevated 2 to 5 fold in GDNF-het and GDNFh animals respectively (Fig. 3d) suggesting that tissue specific miR expression profiles (Fig. 1b) may contribute to differential effects on GDNF transcript and protein levels in diverse tissues.

Next, using in situ hybridization technique, we analyzed GDNF site of expression in GDNFh mice. In the brain this analysis is challenging because GDNF mRNA levels are several orders of magnitude lower compared to e.g. developing kidney and testis where GDNF mRNA can be readily detected in well-defined developmental structures and/or specific cell types (refs). Analysis of developing kidney at E11, where cells in the compartment called CAP condensate of metanephric mesenchyme (CAP-MM) expressing GDNF are sharply bordered with cells what do not express GDNF using a probe recognizing GDNF exons revealed no difference in the site of expression between the genotypes, whereas stronger signal in GDNFh-het and GDNFh mice suggested elevation in GDNF exons containing mRNA levels (Fig. 3e). As expected, an probe recognizing GDNF 3'UTR probe gave weaker signal in GDNFh-het and no,- or in some experiments, close to detection limit signal in GDNFh mice (Fig. 3e, and data not shown), suggesting that like in vitro (Fig. 2 lower panel) the 3'UTR crKO strategy works in vivo (Fig. 3e). Northern blot analysis of GDNF mRNA from developing kidney confirmed elevation of GDNF exons containing mRNA levels and suggested that about 5% of GDNF mRNA derived from the GDNF-3'UTR-crKO allele result from the "read through" of the bGHpA signal resulting in a long ca 7.5 kb transcript containing both the 3'UTR substitution cassette and GDNF wt 3'UTR in the 3' end (Fig. 6g,). Sequence analysis of GDNF transcript further confirmed that GDNF exons are followed by the substitution cassette in GDNFh mice (Fig 6d). Further analysis of GDNF site of expression in GDNFh mice at the tissue or cellular level in E11,5 hind limb and P7 testis respectively revealed no difference from the wt, supporting the conclusion that in GDNFh mice GDNF is expressed by GDNF natively expressing cells (Fig. 6e and f).

### EXAMPLE 5. Development of the brain dopaminergic system in GDNF-3'UTR-crKO (GDNFh) mice

Since "classical" GDNF coding sequence (CDS) knock-out mice have intact brain dopaminergic system at birth but die during P1 due to the lack of kidneys, the role of endogenous GDNF levels in postnatal DA system development has remained obscure (¹⁰, other refs). GDNFh animals enabled us, for the first time, to address the role of endogenous GDNF levels in brain DA system postnatal development and function. First we asked, is the up to 5 fold elevated GDNF in the brain of GDNFh mice biologically active? Towards that end we measured the levels of tyrosine hydroxylase (TH), an enzyme involved in dopamine synthesis, which levels are known to be down regulated 30-70% by exogenous GDNF striatal overexpression ranging from few tens to several hundreds of times over the endogenous GDNF levels, respectively ¹¹⁻¹². QPCR analysis of cDNA derived from rostral brain at P7.5 revealed a similar, 70% downregulation of TH mRNA in 7 day old GDNFh mice (Fig. 4a). Like in the aforementioned studies, no mRNA levels change was observed for other markers of DA metabolism dopamine transporter (DAT) and dopamine decarboxylase (DDC) (data not shown). Next, we measured DA and its metabolite levels in rostral brain at P7 and found that DA and its metabolite homovanillic acid (HVA) levels were significantly increased in GDNFh,- but not in GDNFh-het mice (Fig. 4b, c), indicating a dose dependent response for endogenous GDNF in vivo. As expected, dopamine and its metabolite levels were indistinguishable from the wt in GDNFhr animals (Fig. 4e). Dopamine neurons reside within a midbrain structure called substantia nigra (SN) and project their neuritis into the striatum where GDNF has been hypothesized to act as a target derived neurotrphic factor (NTF) for them after birth (¹⁰, other refs). Next, we counted the number of dopamine neurons in SN using antibodies against two dopamine system markers, TH and vesicular monoamine transporter 2 (VMAT2). We found that the number of TH positive cell bodies in SN pars compacta (SNpc) was increased in both GDNFh-het and GDNFh animals by about 15-20% (Fig. 4f) whereas the number of VMAT2 positive cells was elevated, but not at the statistically significant level (Fig. 4h). No difference in i) optical density of striatal TH immunostaining, or ii) Western blot analysis of TH levels in the rostral brain was observed, indicating no change in the striatal TH protein levels (Fig. 4g and data not shown).

This data suggests that GDNF in GDNF-3'UTR-crKO mice is biologically active, that postnatally GDNF levels regulate brain dopamine system function and that GDNF acts as a target derived NTF for dopamine neurons in SNpc.

### EXAMPLE 6. Uncoupling of renal function from the brain dopamine levels

While GDNFh-het mice were found according to the expected Mendelian ratios at all ages, GDNFh mice were absent upon weaning (Table 1). Anatomical analysis of GDNFh mice revealed hypomorphic kidneys. Kidney size in GDNFh-het mice on the other hand varied from normal to about 5-20% reduced size (Fig. 7a). Further analysis revealed lack of correlation between kidney function and brain dopamine levels both at P7 and in adult GDNFh-het animals (Fig. 7, Table 2). Effects of GDNF 3'UTR/miR regulation on urogenital tract development will be addressed in a manuscript submitted elsewhere.

**Table 1. GDNFh mice die before weaning, GDNFh-het mice are found according to Mendelian expectancy at all ages.**

| | **nr of animals analysed** | **GDNF-het expected** | **GDNF-het found** | **GDNFh expected** | **GDNFh found** |
|---|---|---|---|---|---|
| E10-E18 | 116 | 56 | 62 | 28 | 26 |
| P7.5 | 105 | 62 | 60 | 31 | 14 |
| P22-28 | 22 | 16 | 14 | 8 | 0 |
| 2-4 months | 101 | 48 | 53 | 0 | 0 |

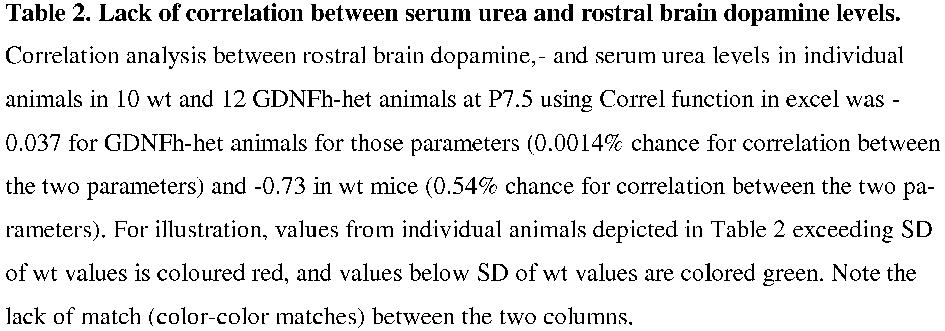

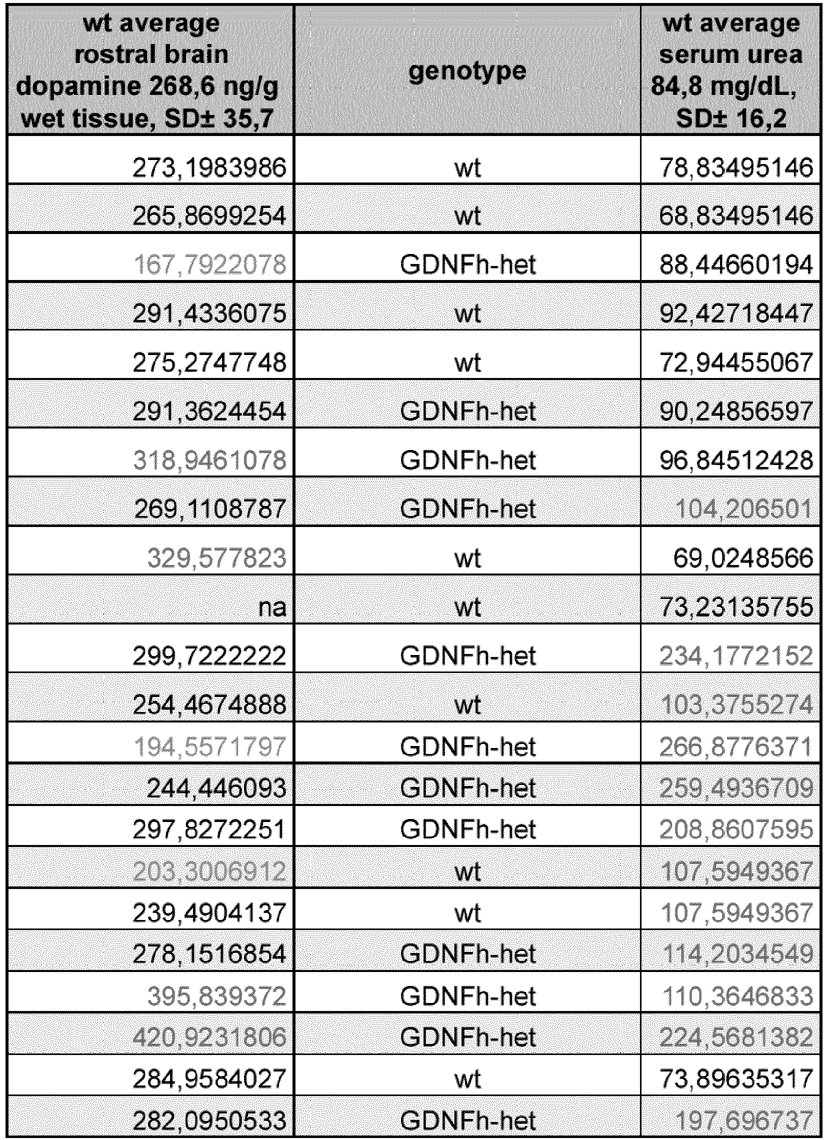

### EXAMPLE 7. Function of the brain dopaminergic system in adult GDNFh-het mice

We analysed GDNF levels and brain dopamine system in adult GDNFh-het animals. Compared to the wt littermate controls, GDNF mRNA and protein levels in adult mice at 2 months of age were upregulated by about 30% in the dorsal striatum and testis (Fig. 5a, b and c), which is less than observed in GDNFh-het mice at P7 (Fig. 3a,b,c,d). However, when in GDNFh-het animals at P7 DA or its metabolite levels were not significantly increased (Fig. 4b,c,d), DA levels in adult GDNF-het mice were elevated both at 2 and 5 months of age (Fig. 5 d). As expected, no difference was observed between adult wt and GDNFhr animals for this parameter (Fig. 5 g). No difference in optical density of striatal TH immunostaining or QPCR analysis of TH mRNA levels in the dorsal striatum was observed at 2 months of age (Fig. 5j and data not shown). This is important, since robust striatal lentivirus mediated GDNF overexpression fails to increase striatal DA levels ¹¹⁻¹². Instead, it leads to profound TH mRNA and protein downregulation between 3 to 6 weeks after virus injection and this downregulation persists at least for 6 months ¹¹⁻¹².
Counting of dopamine neurons at 2 months of age in SNpc revealed a small, but significant ca 13% increase in VMAT2 positive cells (Fig. 5h) and similarly small, but statistically insignificant increase in the number of TH+ cells (Fig. 5i). Finally, we asked whether the aforementioned quantitative differences in the nigrostriatal DA system are also paralleled with functional changes. Towards that end we injected mice with amphetamine, a drug what reverts the direction of synaptic dopamine transporter (DAT), resulting in elevated locomotor activity (¹¹, other refs). We found that compared to wt littermate controls, locomotor activity was enhanced by amphetamine in GDNFh-het mice (Fig. 5k), indicating enhanced dopaminergic transmission. Mice were tested in three independent cohorts by three different experimenters blind regarding the genotypes. All experiments were performed with male mice in 129Sv/C57b16/ICR triple mixed genetic background using gender matched WT littermate controls. Mean values for each cohort separately and pooled (COMB) are shown. P-value of ANOVA between the genotypes (GDNFh-het vs WT) is shown in the last column.

Elevation of endogenous GDNF levels specifically improves motor performance in young mice as shown in Table 3. In addition, elevation of endogenous GDNF levels alleviates age associated decline in motor performance and motor learning in aging mice, as shown in Table 4. Table 5 shows a comparison of phenotypes induced by ectopic GDNF applications versus elevation of endogenous GDNF levels.

**Table 3. Elevation of endogenous GDNF levels specifically improves motor performance in young mice. Mice were tested in three independent cohorts by three different experimenters blind regarding the genotypes. All experiments were performed with male mice in 129Sv/C57b16/ICR triple mixed genetic background using gender matched WT littermate controls. Mean values for each cohort separately and pooled (COMB) are shown. P-value of ANOVA between the genotypes (GDNFh-het vs WT) is shown in the last column. At the beginning of the experimentation, mice were 10 weeks old. Abbreviations: ND - not determined, ACT - spontaneous locomotor activity, RR - accelerating rotarod, VG - vertical grid, CH - coat-hanger, BW - beam walking, GS - grip strength, MR - multiple static rods, PPI - pre-pulse inhibition of acoustic startle reflex, LD-light-dark exploration, HP-hot plate, FST-forced swim test, RI-resident-intruder test, CLAMS-comprehensive laboratory animal monitoring system, metabolic measurements.**

| Age group | YOUNG (Age 10 weeks) | | | | | | | | |
|---|---|---|---|---|---|---|---|---|---|
| Genotype | GDNFh-het | | | | WT | | | | |
| Cohort | coh-1 | coh-2 | coh-3 | COMB | coh-1 | coh-2 | coh-3 | COMB | P-value |
| Number of mice | n=10 | n=9 | n=12 | n=31 | n=10 | n=11 | n=13 | n=34 | |
| Date of beginning | 19-10-2009 | 11-08-2011 | 12-12-2011 | | 19-10-2009 | 11-08-2011 | 12-12-2011 | | |
| Body weight, g | 34.9 | 35.4 | 34.0 | 34.7 | 36.1 | 36.8 | 34.4 | 35.7 | 0.2248 |
| ACT: Distance, cm | 3305 | 3410 | 3231 | 3307 | 3129 | 2722 | 3492 | 3136 | 0.5832 |
| RR: Day1, sec | 130 | 214 | 232 | 194 | 109 | 152 | 173 | 148 | 0.0462 |
| RR: Day2, sec | 207 | 280 | 300 | 264 | 164 | 227 | 220 | 206 | 0.0109 |
| VG: Turn, sec | ND | 4.2 | 5.5 | 5.0 | ND | 10.0 | 4.7 | 7.1 | 0.1125 |
| VG: Fall, sec | ND | 60.0 | 60.0 | 60.0 | ND | 60.0 | 58.5 | 59.2 | 0.3496 |
| GS: Grip, g | 82.8 | 64.3 | 70.5 | 72.7 | 81.3 | 70.1 | 66.0 | 71.5 | 0.7028 |
| MR: Turn, sec | ND | 23.9 | 66.5 | 48.2 | ND | 42.1 | 28.1 | 34.5 | 0.3277 |
| MR: Run, sec | ND | 56.3 | 109.5 | 86.7 | ND | 67.7 | 82.8 | 75.9 | 0.5441 |
| Acoustic startle | 2664 | 3648 | 2909 | 3045 | 2660 | 3665 | 3398 | 3267 | 0.3985 |
| Age group | YOUNG (Age 10 weeks) | | 40.1 | 39.9 | ND | 41.0 | 40.6 | 40.8 | 0.7214 |

| Genotype | GDNFh-het | WT | | | | | | | |
|---|---|---|---|---|---|---|---|---|---|
| Cohort | coh-1 | coh-2 | coh-3 | COMB | coh-1 | coh-2 | coh-3 | COMB | P-value |
| Number of mice | n=10 | n=9 | n=12 | n=31 | n=10 | n=11 | n=13 | n=34 | |
| Date of beginning | 19-10-2009 | 11-08-2011 | 12-12-2011 | | 19-10-2009 | 11-08-2011 | 12-12-2011 | | |
| PPI, % | 53.8 | 39.4 | 43.0 | 45.4 | 63.0 | 45.1 | 35.3 | 46.6 | 0.8514 |
| LD: Time in light, % | 51.4 | ND | ND | | 49.1 | ND | ND | | 0.6695 |
| HP: Latency, sec | 10.2 | ND | ND | | 13.0 | ND | ND | | 0.1314 |
| FST: Immobility, % | 58.1 | ND | ND | | 60.4 | ND | ND | | 0.8060 |
| RI: Social activity, sec | 41.8 | ND | ND | | 45.8 | ND | ND | | 0.5449 |

| CLAMS: | | | | | | | | | |
|---|---|---|---|---|---|---|---|---|---|
| Food intake, g | 11.6 | ND | ND | | 9.8 | ND | ND | | 0.1804 |
| Water intake, ml | 12.8 | ND | ND | | 9.1 | ND | ND | | 0.0582 |
| O2 consumpt., ml/kg/h | 3102 | ND | ND | | 2967 | ND | ND | | 0.5552 |

**Table 4. Elevation of endogenous GDNF levels alleviates age associated decline in motor performance and motor learning in aging mice.**

| Similar to the experiment of Table 3, but at the beginning of the experimentation, mice were 15-17 months old. | | | | | | | | | |
|---|---|---|---|---|---|---|---|---|---|
| Age | OLD (Age 15-17 months) | | | | | | | | |
| Genotype | GDNFh-het | | | | WT | | | | |
| Cohort | coh-1 | coh-2 | coh-3 | COMB | coh-1 | coh-2 | coh-3 | COMB | P-value |
| Number of mice | n=7 | n=4 | n=9 | n=20 | n=7 | n=6 | n=12 | n=25 | |
| Date of beginning | 04-01-2011 | 11-08-2011 | 16-01-2012 | | 04-01-2011 | 11-08-2011 | 16-01-2012 | | |
| Body weight, g | 49.7 | 57.5 | 59.0 | 55.5 | 51.5 | 54.3 | 55.5 | 54.1 | 0.5428 |
| ACT: Distance, cm | 5482 | 4405 | 2413 | 3886 | 3739 | 5490 | 2055 | 3351 | 0.4244 |
| RR: Day1, sec | 101 | 51 | 69 | 76 | 65 | 73 | 56 | 63 | 0.4126 |
| RR: Day2, sec | 161 | 187 | 110 | 143 | 83 | 78 | 89 | 85 | 0.0206 |
| VG: Turn, sec | ND | 9.5 | 3.7 | 5.5 | ND | 23.9 | 15.1 | 18.1 | 0.0064 |
| VG: Fall, sec | 60.0 | 60.0 | 60.0 | 60.0 | 12.1 | 32.8 | 50.8 | 35.6 | 0.0000 |
| CH: Fall, sec | 26.0 | 29.0 | 18.7 | 23.3 | 13.0 | 19.0 | 22.3 | 18.9 | 0.3604 |
| BW: Fall, sec | 18.3 | 30.9 | 33.5 | 27.7 | 24.2 | 37.4 | 45.7 | 37.7 | 0.3490 |
| BW: Crossings, nr | 1.9 | 1.9 | 0.2 | 1.1 | 2.4 | 6.6 | 0.2 | 2.3 | 0.3569 |
| GS: Grip, g | 66.2 | 63.5 | 77.7 | 71.1 | 53.1 | 62.3 | 68.8 | 62.8 | 0.0591 |
| MR: Turn, sec | ND | 29.2 | 128.0 | 97.6 | ND | 25.8 | 127.1 | 93.3 | 0.8632 |
| Acoustic startle | 2542 | 2825 | 818 | 1785 | 1144 | 2491 | 2355 | 2048 | 0.5299 |
| PPI, % | 54.9 | 54.9 | 5.6 | 31.5 | 12.2 | 40.5 | 32.3 | 28.6 | 0.7921 |

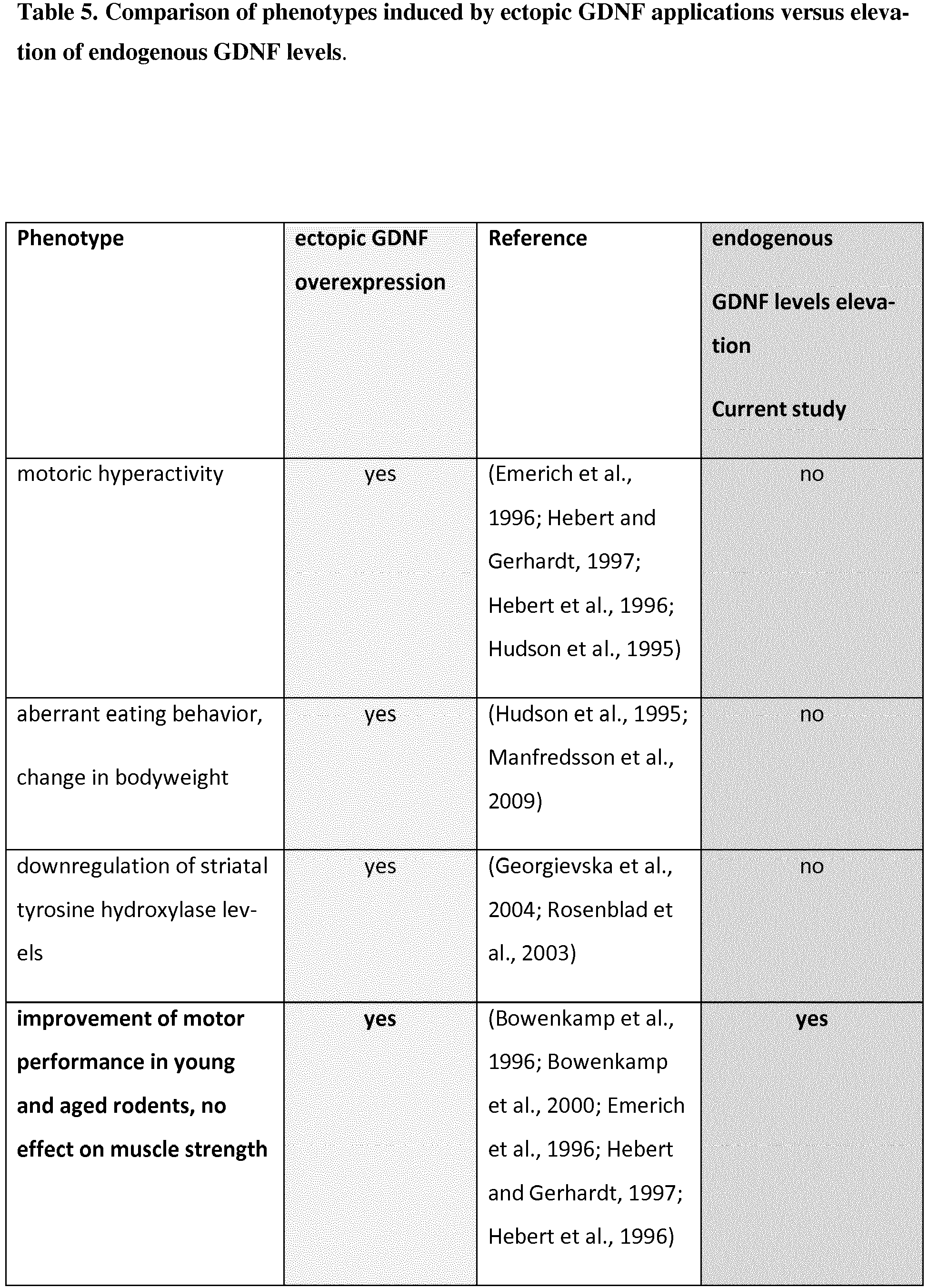

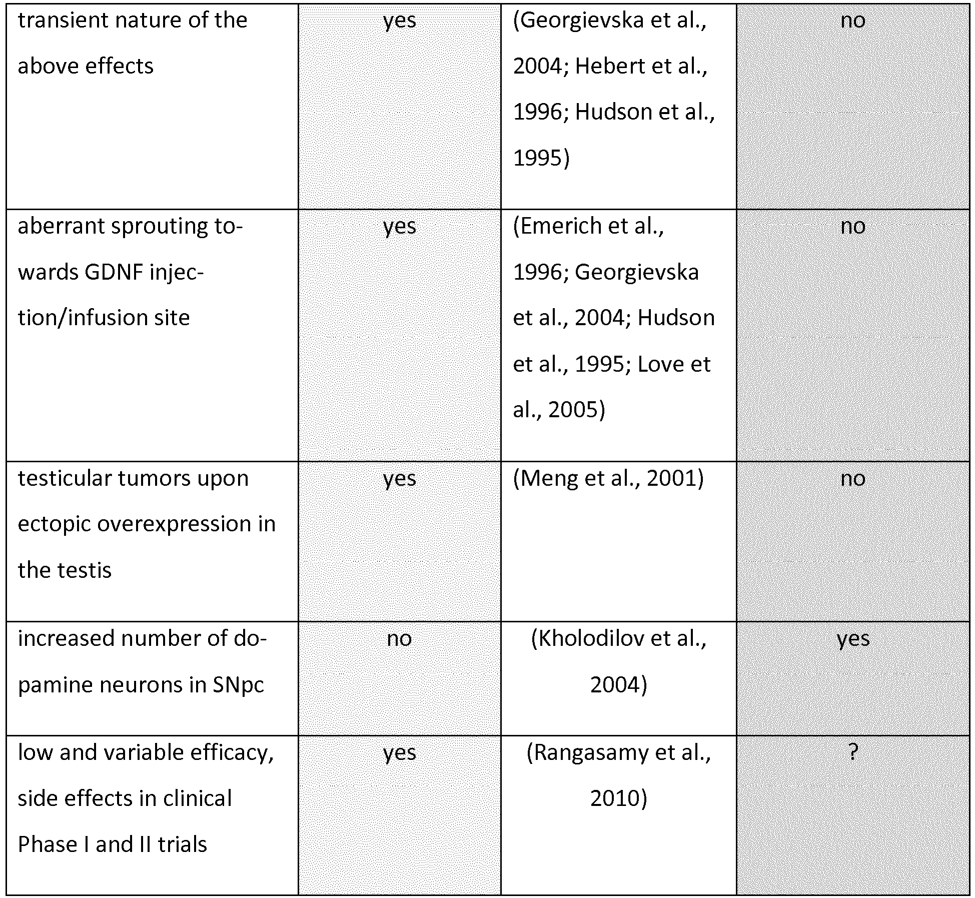

### EXAMPLE 8. Behavioral studies

*Water maze (WM).* The test was introduced for testing spatial learning and memory in rodents (Morris, 1981). The system used by us consisted of a black circular swimming pool (∅ 120 cm) and an escape platform (∅ 10 cm) submerged 0.5 cm under the water surface in the centre of one of four imaginary quadrants. The animals were released to swim in random positions facing the wall and the time to reach the escape platform was measured in every trial. Two training blocks consisting of three trials each were conducted daily. The interval between trials was 4-5 min and between training blocks about 5 hours. The platform remained in a constant location for 3 days (6 sessions) and was thereafter moved to the opposite quadrant for 2 days (4 sessions). The transfer tests were conducted approximately 18 h after the 6^{th} and 10^{th} training sessions. The mice were allowed to swim in the maze for 60 seconds without the platform available and the spatial memory was estimated by the time spent swimming in the quadrant where the platform was located during training. In addition, the swimming distance and the thigmotaxis were measured. Thigmotaxis was defined as the time spent swimming within the outermost ring of the water maze (10 cm from the wall).
After completing the spatial version of the water maze the platform was made visible in the quadrant not employed previously. The mice were tested in one block of three trials (ITI 4-5 min) and the time to reach the platform was measured. The paths of the mice were video-tracked by using a Noldus EthoVision 3.0 system (Noldus Information Technology, Wageningen, The Netherlands). The raw data were analyzed by the same software.

*Rota rod (RR).* For evaluation of coordination and motor learning the accelerating rotarod (Ugo Basile, Comerio, Italy) test was performed on two consecutive days. The mice were given three trials a day with an intertrial interval of 1 hour. Acceleration speed from 4 to 40 r.p.m. over a 5-min period was chosen. The latency to fall off was the measure of motor coordination and improvement across trials was the measure of motor learning. The cut-off time was set at 6 min.

*Beam walking (BW).* The mouse was placed on a horizontal round beam (covered with laboratory tape, outer diameter 2 cm, and length 120 cm, divided into 12 sections and raised to 50 cm above the floor level). The retention time and the number of lines crossed on the beam during 2 min were measured.

*Vertical grid (VG).* The mouse was placed on a horizontal metal grid (22 x 25 cm) raised to 40 cm above the table. The grid was turned vertical with the mouse facing downward. Time until the mouse turned around 180° (cut off 15 sec) or latency to fall was measured.

### EXAMPLE 9. In vivo chronoamperometry

In vivo chronoamperometry was employed to monitor dopamine clearance in the striatum of mice with normal (gdnf*wt*; n=4) and elevated GDNF levels (gdnf+/-; n=4). To perform the recordings, the Fast Analytical Sensing Technology (FAST-16) system (Quanteon, Nicholasville, KY, USA (Hoffman and Gerhard J. Neurochem., 1998; vol 70:179-189) and single carbon fiber electrodes (Quanteon, Nicholasville, KY, USA) were used. This system enables second-by-second quantitative detection of electrochemically active compounds at high spatial resolution. Preceding recordings, the carbon fiber electrodes were coated with nafion (Sigma, Stockholm, Sweden) to increase their selectivity for dopamine (Gerhard et al. Brain Res, 1984; vol. 290:390-395). Nafion is a teflon-derivate that excludes anions such as ascorbic acid, whilst concentrating cations such as the catecholamines at the electrode surface. Electrodes were calibrated in phosphate buffered saline (PBS, 0.05 M, pH=7.4) and ascorbic acid (20 mM) and dopamine (DA-HCl, Sigma, 2 mM) were added during calibration procedure. The electrodes included in this study had selectivity of more than 200:1 for dopamine over ascorbic acid, a limit of detection below 0.05 µM, and responded linearly to dopamine (R² >0.995). Following calibration, the electrode was mounted parallel with a micropipette backfilled with 200uM dopamine (in saline containing 20µM ascorbic acid), at a distance of 130-160µm between the tips. The micropipette was used for application of dopamine during recordings.

Mice were anesthetized with an intraperitonal injection of urethane (∼1.6 g/kg body weight, Sigma, Stockholm, Sweden) and fixed in a stereotaxic frame. Animals were placed on a heating pad to maintain normal body temperature. An incision was made in the scalp and bone overlying the striatum was bilaterally removed using a dental drill. An additional single hole was made caudally for implantation of an Ag/AgCl reference electrode. The electrode/micropipette-assembly was lowered into the striatum stereotactically using a microdrive at +0.3 and +1mm anterior and ±1.8 mm lateral from bregma level. Recordings were performed at two distinct rostrocaudal striatal tracks in each hemisphere. At each recording site, data was collected from four depths below the dura: at -2.0, -2.5, -3.0, and -3.5 mm. The ejected volume was monitored using a scale fitted in the ocular of an operation microscope. Dopamine (10-450nl) was locally applied to evaluate dopamine clearance from the extracellular space. Ejection volumes were varied to produce a range of amplitudes up to 20µM, averaging 5 data points per DV recording site.

During recordings, a square wave potential of 0.55 and 0V (against an Ag/AgCl reference electrode) was applied over the electrode at a frequency of 5 Hz, causing oxidation and subsequent reduction of surrounding analytes. The current produced from the oxidation and reduction reactions were integrated, thus giving an average signal per second for each reaction. Increased extracellular levels of electrochemically active compounds induce a rapid change in the current recorded by the electrode, which is directly proportional to analyte concentration (µM) (Scatton et al. Ann N.Y. Acad. Sci., 1988; vol 537:124-137).

Distinction between the catecholamines was made with red/ox ratios, where previous studies have demonstrated that dopamine has a ratio of 0.8, whilst serotonin for example has 0.2 (Strömberg et al. Exp. Neurol., 1991; vol 112:140-152).

Statistical evaluations between mice with normal (gdnf*wt*) and elevated GDNF levels (gdnf+/-) describe kinetics of dopamine reuptake. Rate (µM/s) describes the concentration of dopamine cleared per second, calculated by multiplying the peak amplitude concentration subtracted from the baseline, using the Michaelis-Menten first order decay constant (k1). All values are presented as means; error bars denote ± standard error of the mean (SEM).

### EXAMPLE 10. Cell culture

Human Embryonic Kidney 293 (HEK-293) cells, Baby Hamster Kidney 21(BHK-21) cells and Human glioblastoma-astrocytoma, epithelial-like cell line(U-87 MG) were cultured at 5% CO₂ and 37° C in cell culture medium containing Dulbecco's Modified Eagle Medium(DMEM, Invitrogen/Gibco) supplemented with 10% Fetal Bovine Serum(FBS) and 100µg/ml Normycin(InvivoGen,USA) . Cells were never allowed to reach a confluency beyond 70% and splitted one day before start of an experiment.

### EXAMPLE 11. Luciferase reporter assay

15000 HEK-293 or 8000 BHK-21 cells per well in 96 well plate (pre-coated with 0.1% gelatin in the case of HEK-293 cells) were seeded one day before transfection. Reporter plasmids were transfected along with Pre-MiRs (Ambion) according to standard protocol recommended for lipofectamine 2000 (Invitrogen). The medium was replaced with fresh cell culture medium after 3-4 hours. The cells were lysed after 24 hours with 1X passive lysis buffer as recommended by the manufacturer (Promega,USA). The luciferase activity was measured with Dual-Luciferase® Reporter Assay System (E1960, Promega) .

### EXAMPLE 12. GDNF mRNA levels measurements with RT PCR

U-87 MG cells were plated on 12 well plates (Cellstar) (0.1X10⁶ cells/ well). Next day, indicated cocktail of pre-miRs (Ambion) or LNA based miR inhibitors (Exiqon) or excess amounts of plasmids containing indicated 3' UTRs acting as miR-sponge along with relevant controls were transfected as recommended by the manufacturer with lipofectamine 2000 (Invitogen). The medium was replaced with fresh cell culture medium after 3-4 hours. The cells were washed with 1X PBS and harvested in TRI Reagent^{®} (Molecular Research Center, USA) after 48 hours and total RNA was isolated. Isolated total RNA was DNAse (Promega) treated and used for reverse transcription (RT) reaction with RevertAid reverse transcriptase (Fermentas). The LightCycler^{®} 480 Real-Time PCR System (Roche Applied Science, USA) was used for quantitative PCR from RT product.

### EXAMPLE 13. ELISA

In order to detect GDNF secreted in the medium by U-87 MG cells, the 0.1X10⁶ cells were plated in 12 well plate (Cellstar) one day before transfection. The cocktail of Pre-miRs (Ambion) were transfected along with controls. The medium was replaced with fresh cell culture medium after 3-4 hours. After 3 hours of recovery, 600µl of serum free OptiMEM supplemented with 0.5 % Bovine serum albumin (BSA) was added to each well. The plate was incubated at 8% CO₂ and 37° for 48 hours and medium was collected and centrifuged at +4°C at 2000rpm for 3 minutes to precipitate cell debris. The GDNF Eₘₐₓ® ImmunoAssay System (Promega,USA) was used according to manufacturer's protocol to detect GDNF protein level in the collected medium.

### EXAMPLE 14. Northern blotting

Total RNA was isolated from mice tissues lysed in TRI Reagent^{®} (Molecular Research Center, USA) as recommended in manufacturer's protocol. The poly A enrichment in samples were carried out using *NucleoTrap*® *mRNA kit (Germany).* The poly A enriched RNAs were run on 1% denatured agarose gel overnight and RNA were detected using DIG based nucleotide detection system (Roche Applied Science, USA).

### EXAMPLE 15. Survival assay

CellTiter-Glo® Luminescent Cell Viability Assay (Promega) was used to determine survival of cells, according to protocol provided by the manusfacturer.

### EXAMPLE 16. Brain dissection

The mice were killed by decapitation, and their brains were rapidly removed from the skull and placed on an ice-cold brain matrix (Stoelting, Wood Dale, IL). Two coronal cuts were made by razor blades at about 1.8 and - 0.2 mm from bregma. From the obtained section, the dorsal striatum was punched below the corpus callosum by using a sample corer (inner diameter, 2 mm). The brains of the P7.5 animals were cut in half with a razor blade at -0.2 mm from bregma and the dorsal part of the brain was collected. Dissected tissue pieces were immediately placed into frozen microcentrifuge tubes and, after weighing, they were stored at - 80°C until assayed.

### EXAMPLE 17. Estimation of monoamines and their metabolites

The brain samples were homogenized in 0.5 ml of homogenization solution consisting of six parts of 0.2 M HCLO4 and one part of antioxidant solution containing oxalic acid in combination with acetic acid and L-cysteine (Kankaanpää et al., 2001). The homogenates were centrifuged at 20,800g for 35 min at 4°C. 300 µl of supernatant was removed to 0.5 ml Vivaspin filter concentrators (10,000 MWCO PES; Vivascience AG, Hannover, Germany) and centrifuged at 8,600g at 4°C for 35 min. Filtrate containing monoamines was analyzed using highpressure liquid chromatography with electrochemical detection. The column (Spherisorb1 ODS2 3 lm, 4.6 3 100 mm2; Waters, Milford, MA) was kept at 45°C with a column heater (Croco-Cil, Bordeaux, France). The mobile phase consisted of 0.1 M NaH2 PO4 buffer, 350 mg/l of octane sulfonic acid, methanol (3.5-5%), and 450 mg/l EDTA, and pH of mobile phase was set to 2.7 using H₃PO₄. Pump (ESA Model 582 Solvent Delivery Module; ESA, Chelmsford, MA) equipped with a pulse damper (SSI LP-21, Scientific Systems, State College, PA) provided 1 ml/min flow rate. Sixty microliters of the filtrate was injected into chromatographic system with a CMA/200 autoinjector (CMA, Stockholm, Sweden). Monoamines and their metabolites were detected using ESA CoulArray Electrode Array Detector, and chromatograms were processed and concentrations of monoamines calculated using CoulArray1 for windows software (ESA, Chelmsford, MA). Monoamine and metabolite values were calculated as nanograms per gram (ng/g) wet weight of tissue.

### EXAMPLE 18. Immunohistochemistry

The mice were anesthetized with sodium pentobarbital (100 mg/kg, i.p.) and perfused intracardially with PBS followed by 4 % paraformaldehyde in 0.1 M sodium phosphate buffer, pH 7.4. The brains were removed, postfixed for 4 h, and stored in sodium phosphate buffer containing 20 % sucrose at 4°C. Coronal striatal and nigral sections were cut and saved individually in serial order at -20°C until used for either tyrosine hydroxylase (TH) or vesicular monoamine transporter 2 (VMAT2) immunostaining.

*TH immunohistochemistry.* The striatal (30 µm) and nigral (40 µm) freefloating sections were stained using standard immunohistochemical procedures. After rinsing with PBS three times for 10 min, sections were quenched with 3% hydrogen peroxide (H₂O₂) and 10% methanol for 5 min and rinsed again in PBS three times for 10 min. Sections were preincubated in 2 % normal goat serum (NGS; Vector Laboratories, Burlingame, CA) and 0.3 % Triton X-100 in PBS for 60 min at room temperature to block nonspecific staining. Thereafter, the sections were incubated with rabbit anti-TH polyclonal antibody (Millipore, Bedford, MA) and diluted 1:2000 in PBS containing NGS (2 %) and Triton X-100 (0.3 %) overnight under gentle shaking. The sections were then rinsed in PBS three times for 10 min and incubated for 2 h with the biotinylated goat anti-rabbit antibody (Vector Laboratories) at 1:200 in PBS containing 0.3 % Triton X-100 at room temperature. The sections were rinsed in PBS three times for 10 min and then the standard avidin- biotin reaction was performed using Vectastain Elite ABC peroxidase kit (Vector Laboratories) following the protocol of the manufacturer. The immunoreactivity was revealed using 0.06 % diaminobenzidine (or 0.025 % for P7,5 sections) and 0.03 % H₂O₂ diluted in PBS and afterward rinsed twice with phosphate buffer and once with PBS. The sections were mounted on gelatin/chrome alume-coated slides, air-dried overnight, dehydrated through graded ethanols, cleared in xylene, and coverslipped with Pertex mounting medium (Cellpath, Hemel Hempstead, UK).

*VMAT2 immunohistochemistry.* Nigral (40 µm) freefloating sections were stained quite similarly as described above for TH. After rinsing with PBS three times for 10 min, sections were quenched with 3% hydrogen peroxide (H₂O₂) and 10% methanol for 15 min and rinsed again in PBS three times for 10 min. Sections were preincubated in 10 % normal horse serum (NHS; Vector Laboratories, Burlingame, CA) and 0.5 % Triton X-100 in PBS for 60 min at room temperature. Thereafter, the sections were incubated with goat anti-VMAT2 polyclonal antibody and diluted 1:4000 in PBS containing NHS (1 %) and Triton X-100 (0.5 %) overnight under gentle shaking. The sections were then rinsed in PBS two times for 10 min, preincubated in 2 % NHS for 15 min and incubated for 2 h with the biotinylated horse anti-goat antibody (Vector Laboratories) at 1:200 in PBS containing 0.5 % Triton X-100 at room temperature. The sections were rinsed in PBS three times for 10 min and then avidin- biotin reaction was performed using Vectastain Elite ABC peroxidase kit (Vector Laboratories) following the protocol suggested by the manufacturer. The immunoreactivity was revealed by first incubating sections for 2 min in 0.012 % diaminobenzidine diluted in PBS and then by adding 10µl of 0.01 % H₂O₂. Afterwards the sections were rinsed twice with phosphate buffer and once with PBS and mounted on gelatin/chrome alume-coated slides, air-dried overnight, dehydrated through graded ethanols, cleared in xylene, and coverslipped with Pertex mounting medium (Cellpath, Hemel Hempstead, UK).

*Striatal densitometry measurements.* Striatal TH-positive fiber staining was assessed by optical density (OD) measurements. Using an Optronics (Goleta, CA) digital camera and a constant illumination table, digitalized images of TH immunostained striatal sections were collected. ODs were measured using Image-Pro Plus software (Version 3.0.1; Media Cybernetics, Silver Spring, MD). For each animal, the OD was measured from three striatal coronal sections and the final reading was calculated as an average of those three values. The nonspecific background correction in each section was done by subtracting the OD value of the corpus callosum from the striatal OD value obtained from the same section. The OD analysis was performed under blinded condition on coded slides.

### EXAMPLE 19. Stereological analysis of TH- and VMAT2-positive cells

The number of TH- and VMAT2-positive neurons in the substantia nigra pars compacta (SNpc) were assessed as described previously (Mijatovic et al., 2007) by a person blinded to the identity of the samples. In brief, TH- and VMAT2-positive cell counts were assessed at medial levels of the SNpc, around the medial terminal nucleus (MTN). TH- and VMAT2-positive somas with brown-stained cytoplasm and bright, round-shaped nucleus were used as the counting unit. From each adult animal, every third section between levels -3.08 and -3.28 mm from the bregma was selected (3 sections per animal). From each P7,5 animal, every second section between levels -2.92 and -3.16 mm from the bregma was selected (3 sections per animal). TH- and VMAT2-positive somas were used as the counting unit. StereoInvestigator (MBF Bioscience, Williston, VT) was used to make outlines of the SNpc, and TH and VMAT2-positive cells were counted within the defined outlines according to optical dissector rules (Gundersen et al., 1988). Cell counts were made at regular predetermined intervals (x = 100 µm; y = 80 µm) within the counting frame (60 µm × 60 µm) superimposed on the image using a 60x oil objective [Olympus BX51 (Olympus Optical, Tokyo, Japan) equipped with an Optronics camera]. The counting frame within the SNpc was positioned randomly by the Stereo Investigator software, thereby creating a systematic random sample of the area. The coefficient of error was calculated as estimate of precision and values <0.1 were accepted.

### EXAMPLE 20. Locomotor activity experiments

To measure amphetamine induced locomotor activity, the mice were individually placed in transparent plastic cages (24 × 24 × 15 cm) with perforated plastic lids placed within activity monitors (open-field activity monitor; MED Associates, St. Albans, GA). The mice were habituated for about 15 min before amphetamine injections. All mice were given D-amphetamine-sulphate (1 mg/kg, i.p; University Pharmacy, Helsinki, Finland). Infrared photobeam interruptions were registered for 60 min immediately after the injections.

### REFERENCES

- 1: Bartel, D. P. MicroRNAs: target recognition and regulatory functions. Cell 136, 215-233 (2009).
- 2: Meng, X. et al. Regulation of cell fate decision of undifferentiated spermatogonia by GDNF. Science 287, 1489-1493 (2000).
- 3: Paratcha, G., Ibanez, C. F. & Ledda, F. GDNF is a chemoattractant factor for neuronal precursor cells in the rostral migratory stream. Mol Cell Neurosci 31, 505-514 (2006).
- 4: Pozas, E. & Ibanez, C. F. GDNF and GFRalphal promote differentiation and tangential migration of cortical GABAergic neurons. Neuron 45, 701-713, doi:S0896-6273(05)00112-1 [pii] 10.1016/j.neuron.2005.01.043 (2005).
- 5: Pascual, A. et al. Absolute requirement of GDNF for adult catecholaminergic neuron survival. Nat Neurosci (2008).
- 6: Landgraf, P. et al. A mammalian microRNA expression atlas based on small RNA library sequencing. Cell 129, 1401-1414 (2007).
- 7: Kim, J. et al. A MicroRNA feedback circuit in midbrain dopamine neurons. Science 317, 1220-1224 (2007).
- 8: Verity, A. N. et al. Differential regulation of glial cell line-derived neurotrophic factor (GDNF) expression in human neuroblastoma and glioblastoma cell lines. J Neurosci Res 55, 187-197, doi:10.1002/(SICI)1097-4547(19990115)55:2<187::AID-JNR6>3.0.CO;2-T [pii] (1999).
- 9: Park, C. Y., Choi, Y. S. & McManus, M. T. Analysis of microRNA knockouts in mice. Hum Mol Genet 19, R169-175, doi:ddq367 [pii] 10.1093/hmg/ddq367 (2010).
- 10: Andressoo, J. O. & Saarma, M. Signalling mechanisms underlying development and maintenance of dopamine neurons. Curr Opin Neurobiol (2008).
- 11: Georgievska, B., Kirik, D. & Bjorklund, A. Overexpression of glial cell line-derived neurotrophic factor using a lentiviral vector induces time- and dose-dependent downregulation of tyrosine hydroxylase in the intact nigrostriatal dopamine system. J Neurosci 24, 6437-6445, doi:10.1523/JNEUROSCI. 1122-04.2004 24/29/6437 [pii] (2004).
- 12: Rosenblad, C., Georgievska, B. & Kirik, D. Long-term striatal overexpression of GDNF selectively downregulates tyrosine hydroxylase in the intact nigrostriatal dopamine system. Eur J Neurosci 17, 260-270, doi:2456 [pii] (2003).
- 13: Andreassi, C. & Riccio, A. To localize or not to localize: mRNA fate is in 3'UTR ends. Trends Cell Biol 19, 465-474, doi:S0962-8924(09)00141-X [pii] 10.1016/j.tcb.2009.06.001 (2009).
- 14: Kramer, E. R. et al. Cooperation between GDNF/Ret and ephrinA/EphA4 signals for motor-axon pathway selection in the limb. Neuron 50, 35-47 (2006).
- 15: Mijatovic, J. et al. Constitutive Ret activity in knock-in multiple endocrine neoplasia type B mice induces profound elevation of brain dopamine concentration via enhanced synthesis and increases the number of TH-positive cells in the substantia nigra. J Neurosci 27, 4799-4809 (2007).

## Claims

1. A short interfering RNA (siRNA), double-stranded RNA (dsRNA), micro-RNA (miRNA), short hairpin RNA (shRNA), miRNA sponge, anti-miRNA, morpholino oligonucleotide, miRNA target site protector, or antisense oligonucleotide targeting miR-133a, miR-133b, miR-125a-5p, miR-125b-5p, miR-30a, miR-30b, miR-96, miR-9, and/or miR-146, for use in the treatment of Parkinson's disease, Alzheimer's disease, Huntington's disease, dementia, ALS, spinal cord injury, age associated memory decline, age related drop in physical activity, or age related decline in motorcoordination, wherein said treatment is **characterized by** increasing the expression level of GDNF in a cell expressing endogenous GDNF.

2. The miRNA sponge of claim 1 for use in the treatment according to claim 1_containing at least one copy of 3'UTR of endogenous GDNF mRNA or a fragment thereof.

## Patentansprüche

1. Kurze interferierende RNA (siRNA, short interfering RNA), doppelsträngige RNA (dsRNA), Mikro-RNA (miRNA), kurze Haarnadel-RNA (shRNA, short hairpin RNA), miRNA-Schwamm, Anti-miRNA, Morpholinoligonukleotid, miRNA-Zielstellen-Protektor oder Antisense-Oligonukleotid gerichtete miR-133a, miR-133b, miR-125a-5p, miR-125b-5p, miR-30a, miR-30b, miR-96, miR-9 und/oder miR-146 zur Verwendung in der Behandlung von Parkinson-Krankheit, Alzheimer-Krankheit, Huntington-Krankheit, Demenz, ALS, Rückenmarkverletzung, altersbedingter kognitiver Leistungsschwäche, altersbedingter Abnahme an körperlicher Aktivität oder altersbedingter Abnahme an Bewegungskoordination, wobei die Behandlung **gekennzeichnet ist durch** eine Erhöhung des Expressionswerts von GDNF in einer Zelle, die endogenen GDNF exprimiert.

2. miRNA-Schwamm nach Anspruch 1 zur Verwendung in der Behandlung nach Anspruch 1, enthaltend mindestens eine Kopie von 3'UTR von endogener GDNF mRNA oder ein Fragment davon.

## Revendications

1. ARN interférent court (ARNsi), ARN double brin (ARNds), micro-ARN (ARNmi), ARN en épingle à cheveux court (ARNsh), éponge d'ARNmi, anti-ARNmi, morpholino oligonucléotide, protecteur de site cible d'ARNmi ou oligonucléotide antisens ciblant Rmi-133a, Rmi-133b, Rmi-125a-5p, Rmi-125b-5p, Rmi-30a, Rmi-30b, Rmi-96, Rmi-9 et/ou Rmi-146 pour utilisation dans le traitement de la maladie de Parkinson, de la maladie d'Alzheimer, de la maladie d'Huntington, de la démence, de la sclérose latérale amyotrophique (ALS), d'une lésion de la moelle épinière, d'un déclin de la mémoire associé à l'âge, d'une chute d'activité physique due à l'âge ou d'un déclin dû à l'âge de la coordination motrice, dans lequel ledit traitement est **caractérisé par** l'augmentation du niveau d'expression du GDNF dans une cellule exprimant le GDNF endogène.

2. Eponge d'ARNmi selon la revendication 1 pour utilisation dans le traitement selon la revendication 1, contenant au moins une copie de 3'UTR d'ARNm de GDNF endogène ou d'un fragment de celui-ci.


### SEQUENCE LISTING

<110> Saarma, Mart
<120> A transgenic knockout animal comprising a heterozygous or homozygous deletion or a functional deletion of the geneâ\200\231s native 3'UTR, a method for producing such animals, and uses of said animals
<160> 2
<170> BiSSAP 1.0
<210> 1
   <211> 2382
   <212> DNA
   <213> Mus musculus
<220>
   <221> SOURCE
   <222> 1..2382
   <223> /mol_type="DNA" /organism="Mus musculus"
<400> 1
   aattctaccg ggtaggggag gcgcttttcc caaggcagtc tggagcatgc gctttagcag 60
   ccccgctggg cacttggcgc tacacaagtg gcctctggcc tcgcacacat tccacatcca 120
   ccggtaggcg ccaaccggct ccgttctttg gtggcccctt cgcgccacct tctactcctc 180
   ccctagtcag gaagttcccc cccgccccgc agctcgcgtc gtgcaggacg tgacaaatgg 240
   aagtagcacg tctcactagt ctcgtgcaga tggacagcac cgctgagcaa tggaagcggg 300
   taggcctttg gggcagcggc caatagcagc tttgctcctt cgctttctgg gctcagaggc 360
   tgggaagggg tgggtccggg ggcgggctca ggggcgggct caggggcggg gcgggcgccc 420
   gaaggtcctc cggaggcccg gcattctgca cgcttcaaaa gcgcacgtct gccgcgctgt 480
   tctcctcttc ctcatctccg ggcctttcga cctgcagcca acgccaccat ggggaccgag 540
   tacaagccca cggtgcgcct cgccacccgc gacgacgtcc cccgggccgt acgcaccctc 600
   gccgccgcgt tcgccgacta ccccgccacg cgccacaccg tcgacccgga ccgccacatc 660
   gagcgggtca ccgagctgca agaactcttc ctcacgcgcg tcgggctcga catcggcaag 720
   gtgtgggtcg cggacgacgg cgccgcggtg gcggtctgga ccacgccgga gagcgtcgaa 780
   gcgggggcgg tgttcgccga gatcggcccg cgcatggccg agttgagcgg ttcccggctg 840
   gccgcgcagc aacagatgga aggcctcctg gcgccgcacc ggcccaagga gcccgcgtgg 900
   ttcctggcca ccgtcggcgt ctcgcccgac caccagggca agggtctggg cagcgccgtc 960
   gtgctccccg gagtggaggc ggccgagcgc gccggggtgc ccgccttcct ggagacctcc 1020
   gcgccccgca acctcccctt ctacgagcgg ctcggcttca ccgtcaccgc cgacgtcgag 1080
   gtgcccgaag gaccgcgcac ctggtgcatg acccgcaagc ccggtgccgg atccatgccc 1140
   acgctactgc gggtttatat agacggtcct cacgggatgg ggaaaaccac caccacgcaa 1200
   ctgctggtgg ccctgggttc gcgcgacgat atcgtctacg tacccgagcc gatgacttac 1260
   tggcaggtgc tgggggcttc cgagacaatc gcgaacatct acaccacaca acaccgcctc 1320
   gaccagggtg agatatcggc cggggacgcg gcggtggtaa tgacaagcgc ccagataaca 1380
   atgggcatgc cttatgccgt gaccgacgcc gttctggctc ctcatatcgg gggggaggct 1440
   gggagctcac atgccccgcc cccggccctc accctcatct tcgaccgcca tcccatcgcc 1500
   gccctcctgt gctacccggc cgcgcgatac cttatgggca gcatgacccc ccaggccgtg 1560
   ctggcgttcg tggccctcat cccgccgacc ttgcccggca caaacatcgt gttgggggcc 1620
   cttccggagg acagacacat cgaccgcctg gccaaacgcc agcgccccgg cgagcggctt 1680
   gacctggcta tgctggccgc gattcgccgc gtttacgggc tgcttgccaa tacggtgcgg 1740
   tatctgcagg gcggcgggtc gtggcgggag gattggggac agctttcggg gacggccgtg 1800
   ccgccccagg gtgccgagcc ccagagcaac gcgggcccac gaccccatat cggggacacg 1860
   ttatttaccc tgtttcgggc ccccgagttg ctggccccca acggcgacct gtacaacgtg 1920
   tttgcctggg ccttggacgt cttggccaaa cgcctccgtc ccatgcacgt ctttatcctg 1980
   gattacgacc aatcgcccgc cggctgccgg gacgccctgc tgcaacttac ctccgggatg 2040
   gtccagaccc acgtcaccac ccccggctcc ataccgacga tctgcgacct ggcgcgcacg 2100
   tttgcccggg agatggggga ggctaactga gctctagagc tcgctgatca gcctcgactg 2160
   tgccttctag ttgccagcca tctgttgttt gcccctcccc cgtgccttcc ttgaccctgg 2220
   aaggtgccac tcccactgtc ctttcctaat aaaatgagga aattgcatcg cattgtctga 2280
   gtaggtgtca ttctattctg gggggtgggg tggggcagga cagcaagggg gaggattggg 2340
   aagacaatag caggcatgct ggggatgcgg tgggctctat gg 2382
<210> 2
   <211> 2566
   <212> DNA
   <213> Mus musculus
<220>
   <221> SOURCE
   <222> 1..2566
   <223> /mol_type="DNA" /organism="Mus musculus"
<400> 2
   gaagttccta ttccgaagtt cctattctct agaaagtata ggaacttcag cttgatatcg      60
   aattaattct accgggtagg ggaggcgctt ttcccaaggc agtctggagc atgcgcttta     120
   gcagccccgc tgggcacttg gcgctacaca agtggcctct ggcctcgcac acattccaca     180
   tccaccggta ggcgccaacc ggctccgttc tttggtggcc ccttcgcgcc accttctact     240
   cctcccctag tcaggaagtt cccccccgcc ccgcagctcg cgtcgtgcag gacgtgacaa     300
   atggaagtag cacgtctcac tagtctcgtg cagatggaca gcaccgctga gcaatggaag 360
   cgggtaggcc tttggggcag cggccaatag cagctttgct ccttcgcttt ctgggctcag 420
   aggctgggaa ggggtgggtc cgggggcggg ctcaggggcg ggctcagggg cggggcgggc 480
   gcccgaaggt cctccggagg cccggcattc tgcacgcttc aaaagcgcac gtctgccgcg 540
   ctgttctcct cttcctcatc tccgggcctt tcgacctgca gccaacgcca ccatggggac 600
   cgagtacaag cccacggtgc gcctcgccac ccgcgacgac gtcccccggg ccgtacgcac 660
   cctcgccgcc gcgttcgccg actaccccgc cacgcgccac accgtcgacc cggaccgcca 720
   catcgagcgg gtcaccgagc tgcaagaact cttcctcacg cgcgtcgggc tcgacatcgg 780
   caaggtgtgg gtcgcggacg acggcgccgc ggtggcggtc tggaccacgc cggagagcgt 840
   cgaagcgggg gcggtgttcg ccgagatcgg cccgcgcatg gccgagttga gcggttcccg 900
   gctggccgcg cagcaacaga tggaaggcct cctggcgccg caccggccca aggagcccgc 960
   gtggttcctg gccaccgtcg gcgtctcgcc cgaccaccag ggcaagggtc tgggcagcgc 1020
   cgtcgtgctc cccggagtgg aggcggccga gcgcgccggg gtgcccgcct tcctggagac 1080
   ctccgcgccc cgcaacctcc ccttctacga gcggctcggc ttcaccgtca ccgccgacgt 1140
   cgaggtgccc gaaggaccgc gcacctggtg catgacccgc aagcccggtg ccggatccat 1200
   gcccacgcta ctgcgggttt atatagacgg tcctcacggg atggggaaaa ccaccaccac 1260
   gcaactgctg gtggccctgg gttcgcgcga cgatatcgtc tacgtacccg agccgatgac 1320
   ttactggcag gtgctggggg cttccgagac aatcgcgaac atctacacca cacaacaccg 1380
   cctcgaccag ggtgagatat cggccgggga cgcggcggtg gtaatgacaa gcgcccagat 1440
   aacaatgggc atgccttatg ccgtgaccga cgccgttctg gctcctcata tcggggggga 1500
   ggctgggagc tcacatgccc cgcccccggc cctcaccctc atcttcgacc gccatcccat 1560
   cgccgccctc ctgtgctacc cggccgcgcg ataccttatg ggcagcatga ccccccaggc 1620
   cgtgctggcg ttcgtggccc tcatcccgcc gaccttgccc ggcacaaaca tcgtgttggg 1680
   ggcccttccg gaggacagac acatcgaccg cctggccaaa cgccagcgcc ccggcgagcg 1740
   gcttgacctg gctatgctgg ccgcgattcg ccgcgtttac gggctgcttg ccaatacggt 1800
   gcggtatctg cagggcggcg ggtcgtggcg ggaggattgg ggacagcttt cggggacggc 1860
   cgtgccgccc cagggtgccg agccccagag caacgcgggc ccacgacccc atatcgggga 1920
   cacgttattt accctgtttc gggcccccga gttgctggcc cccaacggcg acctgtacaa 1980
   cgtgtttgcc tgggccttgg acgtcttggc caaacgcctc cgtcccatgc acgtctttat 2040
   cctggattac gaccaatcgc ccgccggctg ccgggacgcc ctgctgcaac ttacctccgg 2100
   gatggtccag acccacgtca ccacccccgg ctccataccg acgatctgcg acctggcgcg 2160
   cacgtttgcc cgggagatgg gggaggctaa ctgagctcta gagctcgctg atcagcctcg    2220
   actgtgcctt ctagttgcca gccatctgtt gtttgcccct cccccgtgcc ttccttgacc    2280
   ctggaaggtg ccactcccac tgtcctttcc taataaaatg aggaaattgc atcgcattgt    2340
   ctgagtaggt gtcattctat tctggggggt ggggtggggc aggacagcaa gggggaggat    2400
   tgggaagaca atagcaggca tgctggggat gcggtgggct ctatggcttc tgaggcggaa    2460
   agaaccagct ggggctcgag atccactagt tctagcctcg aggctagagc ggccggccga    2520
   agttcctatt ccgaagttcc tattctctag aaagtatagg aacttc                   2566